(19) Europäisches Patentamt
European Patent Office
Office européen des brevets

(11) EP 4 166 549 A1

(12) EUROPEAN PATENT APPLICATION
published in accordance with Art. 153(4) EPC

(43) Date of publication:
**19.04.2023 Bulletin 2023/16**

(21) Application number: **21816791.4**

(22) Date of filing: **07.06.2021**

(51) International Patent Classification (IPC):
***C07D 401/04*** *(2006.01)* ***C07D 401/14*** *(2006.01)*
***C07D 471/10*** *(2006.01)* ***C07D 487/04*** *(2006.01)*
***A61P 35/00*** *(2006.01)*

(52) Cooperative Patent Classification (CPC):
**A61K 31/519; A61P 35/00; A61P 35/02; C07D 401/04; C07D 401/14; C07D 471/10; C07D 487/04; C07D 519/00**

(86) International application number:
**PCT/CN2021/098604**

(87) International publication number:
**WO 2021/244659 (09.12.2021 Gazette 2021/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.06.2020 CN 202010505753**

(71) Applicant: **Etern Biopharma (Shanghai) Co., Ltd.**
**Shanghai 201210 (CN)**

(72) Inventor: **ZHENG, Qiangang**
**Shanghai 201210 (CN)**

(74) Representative: **Santoro, Sofia et al**
**Società Italiana Brevetti S.p.A.**
**Via Giosuè Carducci, 8**
**20123 Milano (IT)**

# (54) ISOTOPE-SUBSTITUTED SPIRO AROMATIC RING COMPOUND AND APPLICATION THEREOF

(57) The present invention provides an isotope-substituted spiro aromatic ring compound represented by the following formula I, or a pharmaceutically acceptable salt thereof, or an enantiomer, a diastereomer, a tautomer, a solvate, a polymorph, a prodrug or a metabolite thereof. Also provided are a preparation method for the compound and use thereof. Compared with isotope-unsubstituted compounds, the isotope-substituted compound of the present invention has a longer half-life, a higher plasma concentration, and more excellent pharmacokinetic properties.

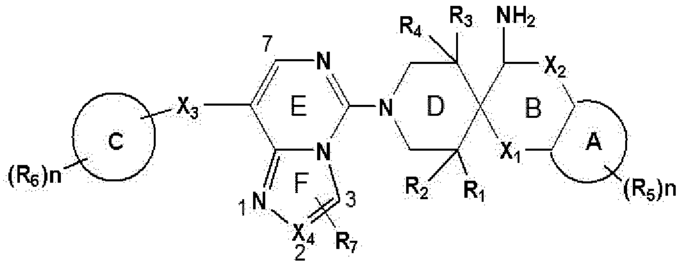

I



Processed by Luminess, 75001 PARIS (FR)

## Description

## Technical Field

The present disclosure relates to the field of spiro aromatic ring compound, in particular, to an isotope-substituted spiro aromatic ring compound that may be used for SHP2 inhibitors, a pharmaceutically acceptable salt thereof, or an enantiomer, a diastereomer, a tautomer, a solvate, a prodrug, or a metabolite thereof. Further, the present disclosure also relates to a method of preparing said compounds, pharmaceutical compositions containing said compounds, and the use of said compounds for the preparation of drugs for the treatment of diseases or disorders associated with abnormal SHP2 activity.

Background

Protein tyrosine phosphatase SHP2, which occupies an extremely important position in the cell signaling process, is a target for the development of treatments for major diseases such as diabetes, autoimmune diseases and cancers, etc. SHP2 is mutated or highly expressed in a variety of diseases, such as Noonan Syndrome, Leopard Syndrome, adolescent myelomonocytic leukemia, neuroblastoma, melanoma, acute myeloid leukemia, breast cancer, esophageal cancer, lung cancer, colon cancer, head cancer, neuroblastoma, head and neck squamous cell carcinoma, stomach cancer, anaplastic large cell lymphoma and glioblastoma and so on. Molecular biology studies have shown that SHP2 is involved in multiple tumor cell signaling pathways, such as MAPK, JAK/STAT, and PI3K/Akt, etc. SHP2 is also responsible for signaling the PD1-PDL1 immunosuppressive pathway. Thus, inhibiting the activity of SHP2 is able to reverse immunosuppression in the tumor microenvironment.

SHP2 consists of two N-terminal Src homologous 2 domains (N-SH2 and C-SH2) and a protein tyrosine phosphatase catalytic domain (PTP). In the self-inhibiting state, N-SH2 binds to PTP to form a circular structure, thereby hindering the binding of PTP to the substrate, so that the enzyme catalytic activity is inhibited. When the tyrosine of the upstream receptor protein is phosphorylated, it binds to N-SH2 and PTP catalytic domain is released to exert phosphatase activity.

At present, the development of SHP2 inhibitors is mainly based on allosteric inhibitors in the non-catalytic region, such as some compounds disclosed in WO2015107493A1, WO2016203404A1, WO2016203406A1, WO2017216706A1, WO2017211303A1, CN201710062495, WO2018136265A1, WO2018057884 and so on. It is shown in the study of this year that SHP2 has attracted increasing attention as a novel druggable target. Therefore, there is an urgent need in the field to develop SHP2 inhibitors with novel structure, good biological activity and high druggability.

Summary

The object of the present disclosure is to provide an isotope-substituted compound represented by Formula I or a pharmaceutically acceptable salt thereof, a pharmaceutical composition comprising the compound or a pharmaceutically acceptable salt thereof, and the use of the compound or the pharmaceutical composition thereof in the prevention and treatment of diseases or disorders associated with SHP2 abnormalities.

A first aspect of the present disclosure provides an isotope-substituted compound represented by formula I, or a pharmaceutically acceptable salt thereof, or an enantiomer, a diastereomer, a tautomer, a solvate, a polymorph, a prodrug, or a metabolite thereof,

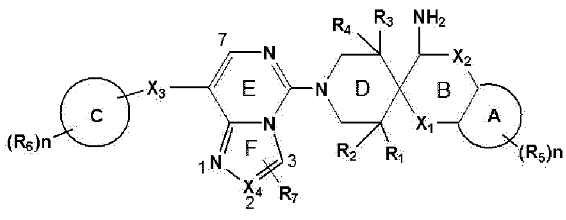

I

,

wherein,

$X_1$ and $X_2$ are each independently selected from a bond, O, $CR_aR_b$, or $NR_c$;
$X_3$ is selected from a bond, $CR_aR_b$, $NR_c$, S or O;

$X_4$ is selected from N or $CR_c$;

$R_a$, $R_b$ and $R_c$ are each independently selected from H, D, halogen, substituted or unsubstituted $C_{1-6}$ alkyl, or substituted or unsubstituted $C_{1-6}$ alkoxyl;

$R_1$, $R_2$, $R_3$, $R_4$ and $R_7$ are each independently selected from H, D, -OH, halogen, substituted or unsubstituted amino, substituted or unsubstituted $C_{1-6}$ alkyl, or substituted or unsubstituted $C_{1-6}$ alkoxyl; and cannot be -OH or $-NH_2$ at the same time;

ring A is selected from substituted or unsubstituted $C_{4-8}$ cyclic hydrocarbyl, substituted or unsubstituted 4-8 membered heterocyclyl, substituted or unsubstituted $C_{5-10}$ aryl groups, or substituted or unsubstituted 5-10 membered heteroaryl, wherein the heterocyclyl or heteroaryl comprises 1-3 heteroatoms selected from the group consisting of N, O, S and P;

ring C is selected from substituted or unsubstituted $C_{4-8}$ cyclic hydrocarbyl, substituted or unsubstituted 5-6 membered monocyclic heterocyclyl, substituted or unsubstituted 8-10 membered bicyclic heterocyclyl, substituted or unsubstituted C5-10 monocyclic or bicyclic aryl, substituted or unsubstituted 5-6 membered monocyclic heteroaryl, or substituted or unsubstituted 8-10 membered bicyclic heteroaryl, wherein the heterocyclyl or heteroaryl comprise 1-4 heteroatoms selected from: N, O, S or P;

$R_5$ and $R_6$ are independently selected from H, D, -OH, halogen, cyano, $-NO_2$, substituted or unsubstituted amino, substituted or unsubstituted $C_{1-6}$ alkyl, or substituted or unsubstituted $C_{1-6}$ alkoxyl;

n is any integer from 0 to 3; and

wherein the substitution refers to one or more hydrogen atoms on the group is substituted by a substituent selected from the group consisting of halogen, -OH, $-NO_2$, $-NH_2$, -NH (unsubstituted or halogenated $C_{1-6}$ alkyl), -N(unsubstituted or halogenated $C_{1-6}$ alkyl)$_2$, -CN, unsubstituted or halogenated $C_{1-8}$ alkyl, unsubstituted or halogenated $C_{1-8}$ alkoxyl, unsubstituted or halogenated $C_{1-8}$ alkoxyl-$C_{1-8}$ alkyl, unsubstituted or halogenated $C_{3-8}$ cycloalkyl-$C_{1-8}$ alkyl, unsubstituted or halogenated $C_{1-6}$ alkylcarbonyl, unsubstituted or halogenated $C_{1-6}$ alkoxylcarbonyl, hydroxamic acid group, unsubstituted or halogenated $C_{1-6}$ alkyl mercapto, $-S(O)_2N$ (unsubstituted or halogenated $C_{1-6}$ alkyl)$_2$, $-S(O)_2$ unsubstituted or halogenated $C_{1-6}$ alkyl, -N(unsubstituted or halogenated $C_{1-6}$ alkyl)$S(O)_2N$(unsubstituted or halogenated $C_{1-6}$ alkyl)$_2$, -S(O)N(unsubstituted or halogenated $C_{1-6}$ alkyl)$_2$, -S(O)(unsubstituted or halogenated $C_{1-6}$ alkyl), -N(unsubstituted or halogenated $C_{1-6}$ alkyl)S(O)N(unsubstituted or halogenated $C_{1-6}$ alkyl)$_2$, -N (unsubstituted or halogenated $C_{1-6}$ alkyl)S(O)(unsubstituted or halogenated $C_{1-6}$ alkyl), unsubstituted or halogenated $C_{5-10}$ aryl, unsubstituted or halogenated 5-10 membered heteroaryl, unsubstituted or halogenated $C_{4-8}$ cyclic hydrocarbyl, or unsubstituted or halogenated 4-8 membered heterocyclyl, wherein the heterocyclic group and the heteroaryl comprise 1-4 heteroatoms selected from: N, O or S;

wherein the isotope-substitution refers to one or more ring carbon atoms in one or more rings of ring A, ring B, ring C, ring D, ring E and ring F are substituted with $^{13}C$, and/or hydrogen atoms on one or more ring atoms in one or more rings of ring A, ring B, ring C, ring D, ring E and ring F are substituted with deuterium.

Herein, $R_1$-$R_4$ and $R_7$ "cannot be -OH or $-NH_2$ at the same time" as described in the definition means that $R_1$ and $R_2$ cannot be both -OH or $-NH_2$, $R_3$ and $R_4$ cannot be both -OH or $-NH_2$.

As a preferred embodiment, in compound of formula I, hydrogen atoms on one or more ring atoms of one or more rings in ring A, ring B, ring C, ring E and ring F are substituted with deuterium. Further preferably, in compound of formula I, hydrogen atoms on one or more ring atoms of ring B are substituted with deuterium; preferably, 1-3 hydrogen atoms on one or more ring atoms of ring B are substituted with deuterium.

As a preferred embodiment, the isotope-substitution is deuterated, wherein hydrogen atoms at one or more positions selected from the following positions are deuterated: hydrogen atoms on the ring atom at any positions other than heterocyclic atoms in ring A; and/or, hydrogen atoms on the ring atoms substituted with amino in ring B, and/or, hydrogen atoms on $X_1$ and/or $X_2$; and/or, hydrogen atoms on the ring atom at any positions other than heterocyclic atom in ring C; and/or, hydrogen atoms on the ring atom at 7-position in ring E; and/or, hydrogen atoms on the ring atoms at 2- and 3-positions in ring F.

As a preferred embodiment, the isotope-substitution is deuterated, wherein 1 to 4 hydrogen atoms are deuterated.

As a preferred embodiment, the isotope-substitution is deuterated, wherein hydrogen atoms on the ring atoms at 7-position of the ring E and/or hydrogen atoms on the ring atoms at 2- and 3-positions of the ring F are deuterated.

As a preferred embodiment, ring C is pyridinyl (preferably pyridin-4-yl) or phenyl, the isotope-substitution is deuterated at one or more positions that are not substituted by $R_6$. Preferably, the ring C is pyridyl, the isotope-substitution is deuterated at one or more positions that are not substituted by $R_6$.

As a preferred embodiment, ring A is a pyridine ring, the isotope-substitution is deuterated at one or more positions that are not ring atoms in addition to the pyridine ring nitrogen atom.

As a preferred embodiment, ring A is a pyridine ring, ring C is pyridin-4-yl or phenyl, $X_4$ is CH, the isotope-substitution is deuterated at one or more rings of ring A, ring C, ring E and ring F; preferably, the total number of deuterium

substitution in the compound is 1-4. As a preferred embodiment, ring A is a pyridine ring, Ring C is pyridin-4-yl, $X_4$ is CH, the isotope-substitution is deuterated on ring A, ring C, ring E or ring F; preferably, the total number of deuterium substitution in the compound is 1-4.

As a preferred embodiment, one of $X_1$ and $X_2$ is $CH_2$, and the other is a bond.

As a preferred embodiment, $X_3$ is S.

As a preferred embodiment, $X_4$ is selected from N or CH.

As a preferred embodiment, $R_1$, $R_2$, $R_3$, $R_4$ and $R_7$ are each independently selected from H, D, -OH, -F, -Cl, -Br,-$NH_2$, -$NHC_{1-3}$ alkyl, methyl, ethyl, propyl, isopropyl, butyl, methoxy, ethoxy, propoxy, isopropoxy, $C_{1-3}$ alkyl that is substituted with halogen, -$NH_2$, -OH, $C_{1-3}$ alkyl or $C_{1-3}$ alkoxyl, or $C_{1-3}$ alkoxyl that is substituted with halogen, -$NH_2$, -OH, $C_{1-3}$ alkyl or $C_{1-3}$ alkoxyl; and $R_1$ and $R_2$ cannot be -OH or -$NH_2$ at the same time, and $R_3$ and $R_4$ cannot be -OH or -$NH_2$ at the same time.

As a preferred embodiment, $R_5$ and $R_6$ are each independently selected from H, D, -OH, -F, -Cl, -Br, -CN, -$NH_2$, -$NHC_{1-3}$ alkyl, methyl, ethyl, propyl, isopropyl, butyl, methoxy, ethoxy, propoxy, isopropoxy, $C_{1-3}$ alkyl that is substituted with halogen, -$NH_2$, -OH, $C_{1-3}$ alkyl or $C_{1-3}$ alkoxyl, or $C_{1-3}$ alkoxyl that is substituted with halogen, -$NH_2$, -OH, $C_{1-3}$ alkyl or $C_{1-3}$ alkoxyl.

As a preferred embodiment, the substituent is selected from -F, -Cl, -Br, -OH, -$NO_2$, -$NH_2$, -NH($C_{1-6}$ alkyl), -N($C_{1-6}$ alkyl)$_2$, -CN, $C_{1-6}$ alkyl, $C_{1-4}$ alkoxyl, $C_{1-4}$ alkoxyl-$C_{1-6}$ alkyl, $C_{3-8}$ cycloalkyl-$C_{1-8}$ alkyl, $C_{1-6}$ alkyl carbonyl, $C_{1-6}$ alkoxylcarbonyl, $C_{1-6}$ alkyl mercapto, -$S(O)_2N$($C_{1-6}$ alkyl)$_2$, -$S(O)_2C_{1-6}$ alkyl, -N($C_{1-6}$ alkyl)$S(O)_2N$($C_{1-6}$ alkyl)$_2$, -S(O)N($C_{1-6}$ alkyl)$_2$, -S(O)($C_{1-6}$ alkyl), -N($C_{1-6}$ alkyl)S(O)N($C_{1-6}$ alkyl)$_2$, -N($C_{1-6}$ alkyl)S(O)($C_{1-6}$ alkyl), substituted or unsubstituted $C_{5-10}$ aryl, substituted or unsubstituted 5-10 membered heteroaryl, substituted or unsubstituted $C_{4-8}$ cyclic hydrocarbyl, or substituted or unsubstituted 4-8 membered heterocyclyl, wherein the heterocyclic group and heteroaryl comprise 1-4 heteroatoms selected from the group consisting of N, O and S.

As a preferred embodiment, the substituent is selected from -F, -Cl, -Br, -OH, -$NO_2$, -$NH_2$, -NH($C_{1-3}$ alkyl), -N($C_{1-3}$ alkyl)$_2$, -CN, $C_{1-3}$ alkyl, $C_{1-3}$ alkoxyl, $C_{1-3}$ alkylcarbonyl, cyclopropyl, cyclobutyl, cyclopentyl, cyclopentenyl, cyclohexyl, cyclhexenyl, cyclhexadienyl, cycloheptyl, cyclooctyl, pyrrolidinyl, morpholinyl, piperazinyl, homopiperazinyl, piperidinyl, thiomorpholinyl, phenyl, naphthyl, anthryl, phenanthryl, fluorenyl, thienyl, imidazolyl, pyrazoyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furyl, pyrrolyl, triazolyl, tetrazolyl, triazinyl, indolizinyl, isoindolyl, indazolyl, isoindazolyl, purinyl, quinolyl, or isoquinolyl.

As a preferred embodiment, the substituent is selected from -F, -Cl, -Br, -OH, -$NO_2$, -$NH_2$, -NH($C_{1-3}$ alkyl), -N($C_{1-3}$ alkyl)$_2$, -CN, methyl, ethyl, propyl, isopropyl, butyl, methoxy, ethoxy, propoxy, isopropoxy or phenyl.

As a preferred embodiment, the ring C is:

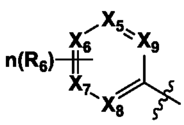 , 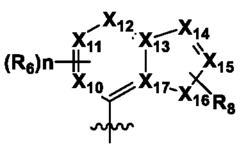 or 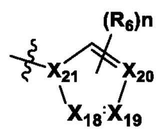 ;

wherein,

$X_5$, $X_6$, $X_7$, $X_8$ and $X_9$ are each independently selected from N or $CR_d$; and at most three of them are N at the same time;
$X_{10}$, $X_{11}$, $X_{12}$, $X_{13}$, $X_{14}$, $X_{15}$, $X_{16}$ and $X_{17}$ are each independently selected from N or $CR_d$; and at most five of them are N at the same time;
$X_{18}$, $X_{19}$, $X_{20}$ and $X_{21}$ are each independently selected from N or $CR_d$; and at most three of them are N at the same time;
$R_6$ and $R_8$ are each independently selected from H, D, -$NH_2$, -CN, -OH, -$NO_2$, halogen, unsubstituted or halogenated $C_{1-6}$ alkyl, or unsubstituted or halogenated $C_{1-6}$ alkoxyl; and
said $R_d$ is selected from H, D, halogen, unsubstituted or halogenated $C_{1-6}$ alkyl, or unsubstituted or halogenated $C_{1-6}$ alkoxyl;
wherein the wavy line indicates the position where ring C and $X_3$ are connected.

As a preferred embodiment, the ring C is:

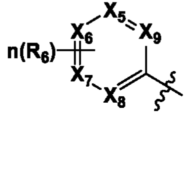 or 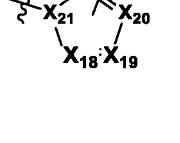

wherein,

0, 1 or 2 of $X_5$, $X_6$, $X_7$, $X_8$ and $X_9$ are N and the rest are $CR_d$;
0, 1 or 2 of $X_{18}$, $X_{19}$, $X_{20}$ and $X_{21}$ are N and the rest are $CR_d$;
$R_6$ is selected from H, D, $-NH_2$, -CN, -OH, $-NO_2$, -F, -Cl, -Br, methyl, ethyl, propyl, isopropyl, butyl, methoxy, ethoxy, propoxy, isopropoxy, fluorinated or brominated $C_{1-3}$ alkyl, or fluorinated or brominated $C_{1-3}$ alkoxyl; and
said $R_d$ is selected from H, D, -F, -Cl, -Br, methyl, ethyl, propyl, isopropyl, butyl, methoxy, ethoxy, propoxy, isopropoxy, fluorinated or brominated $C_{1-3}$ alkyl, or fluorinated or brominated $C_{1-3}$ alkoxyl;
wherein the wavy line indicates the position where ring C and $X_3$ are connected.

As a preferred embodiment, the ring C is:

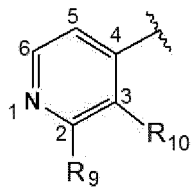

;

wherein $R_9$ and $R_{10}$ are each independently H, halogen, -NR'R" or unsubstituted $C_{1-6}$ alkyl groups, where R' and R" are each independently H or $C_{1-4}$ alkyl; preferably, $R_9$ is -NR'R", $R_{10}$ is halogen.

As a preferred embodiment, the ring C is:

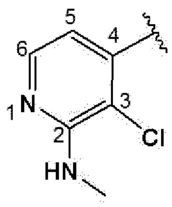

,

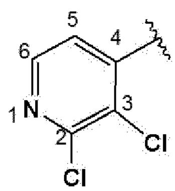

or

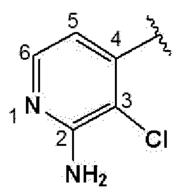

;

preferably, hydrogen atoms at 5- and/or 6- positions of the pyridine ring are substituted with deuterium.

As a preferred embodiment, the ring C is phenyl substituted with halogen atoms at 2- and 3- positions, or 5- and 6- positions, preferably phenyl substituted with 2 chlorine atom; wherein one or more hydrogen atoms on the ring carbon atoms that are not substituted with halogen of phenyl are substituted with D, preferably one hydrogen atom is substituted with D.

As a preferred embodiment, the ring C is:

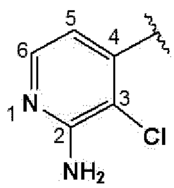

wherein the hydrogen atoms at 5- and/or 6- positions are not substituted with deuterium, or 1-3 hydrogen atoms at 5- and/or 6- positions are substituted with deuterium.

As a preferred embodiment, the ring A is selected from substituted or unsubstituted $C_{4-6}$ cyclic hydrocarbyl, substituted or unsubstituted 4-6 membered heterocyclic group, substituted or unsubstituted $C_{5-6}$ aryl, or substituted or unsubstituted 5-6 membered heteroaryl, wherein the heterocyclic group or heteroaryl comprise 1-3 N atoms.

As a preferred embodiment, the ring A is selected from any one of the following group consisting of:

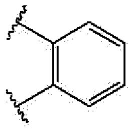, 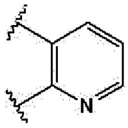, 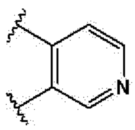, 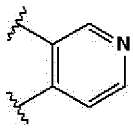, 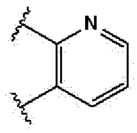, 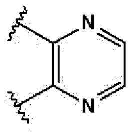,

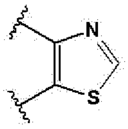, 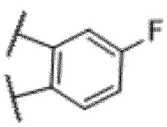, 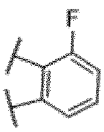, and 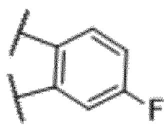;

wherein the wavy line represents the position of ring A fused with ring B, preferably, one or more hydrogen atoms at the ring atom positions other than the ring nitrogen atom and the ring atom positions that are substituted with F in the ring A are substituted by D.

As a preferred embodiment, the ring A is any one selected from the group consisting of:

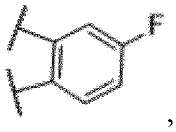, 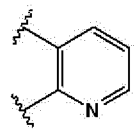, 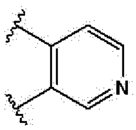, 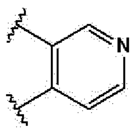, 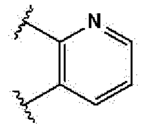, and 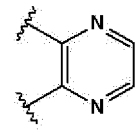;

wherein one or more hydrogen atoms at the ring atom positions other than the ring nitrogen atom and the ring atom positions that are substituted with F in the ring A are substituted by D.

As a preferred embodiment, the ring A is

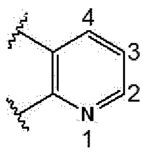,

wherein the isotope-substitution is deuterated at 2-, 3- and/or 4-positions.

As a further preferred embodiment, the compound has a structure selected from the group consisting of:

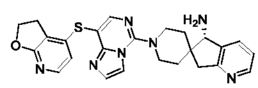 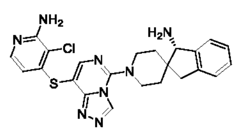 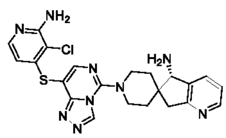 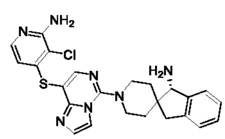 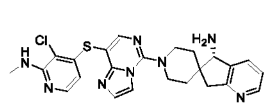

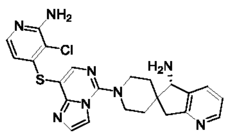 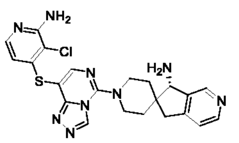 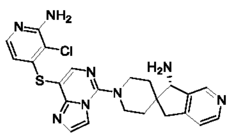 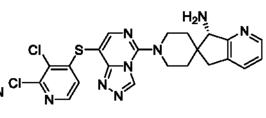 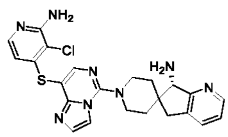

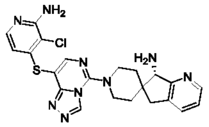 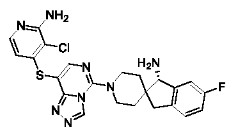 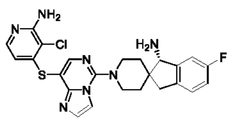 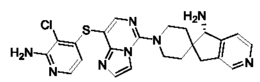 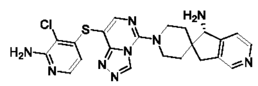

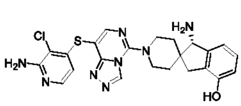 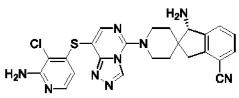 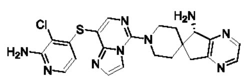 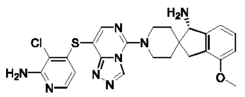 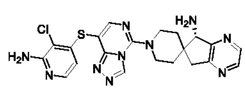

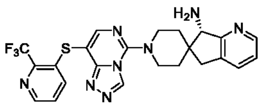 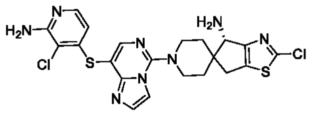 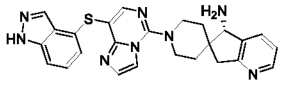 ;

wherein hydrogen atoms on one or more rings of the compound are substituted by D with the substitution position as described in any of the preceding embodiments.

As a further preferred embodiment, the present disclosure provides an isotope-substituted compound represented by formula I, a pharmaceutically acceptable salt thereof, or a enantiomer, a diastereomer, a tautomer, a solvate, a polymorph, a prodrug or metabolite thereof, wherein:

the ring A is:

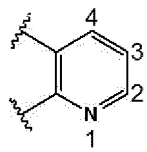

,

where the wavy line represents the position of ring A fused with ring B;
wherein, in the ring B, one of $X_1$ and $X_2$ is a bond and the other is $CH_2$;
$X_3$ is S or O, preferably S;
$X_4$ is CH;
$R_1$ and $R_3$ are each independently H and unsubstituted $C_{1-6}$ alkyl, preferably both are H;
$R_2$ and $R_4$ are each independently H and unsubstituted $C_{1-6}$ alkyl, preferably both are H;
$R_7$ is H, hydroxyl, halogen and unsubstituted $C_{1-6}$ alkyl, preferably H or $C_{1-4}$ alkyl;
the ring C is:

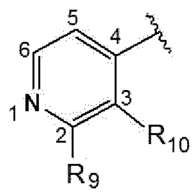

,

wherein $R_9$ and $R_{10}$ are each independently H, halogen, amino or unsubstituted $C_{1-6}$ alkyl, preferably are each independently halogen, amino or $C_{1-6}$ alkyl; more preferably, $R_9$ and $R_{10}$ are both halogens, or one of $R_9$ and $R_{10}$ is a halogen and the other is $C_{1-6}$ alkyl, or $R_9$ is amino, $R_{10}$ is a halogen; where the wavy line represents the position where ring C is connected to $X_3$;
wherein the isotope substation means 1-4 hydrogen atoms on the ring atom are replaced by deuterium, wherein the hydrogen atoms that are substituted with deuterium are selected from one or more of the following:

hydrogen atoms at 2-, 3-, 7- positions in imidazo[1,2-c]pyrimidine ring; and/or
hydrogen atoms at 5-, 6- positions when ring C is a pyridine ring; and/or
hydrogen atoms at 2-, 3-, 4- positions in ring A; and/or
hydrogen atoms on the carbon atoms substituted with amino in ring B, and/or hydrogen atoms attached to $X_1$ and $X_2$ when they are not a bond; preferably hydrogen atoms on the carbon atoms substituted with amino in ring B, or one or two of one or two hydrogen atoms attached to $X_1$ and $X_2$ when they are not a bond and hydrogen atoms on the carbon atoms substituted with amino.

As a further preferred embodiment, the compound has a structure selected from the group consisting of:

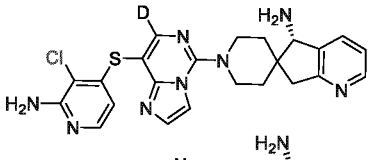
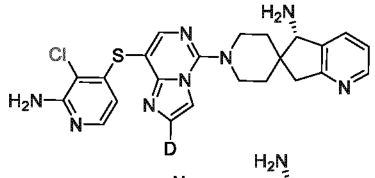
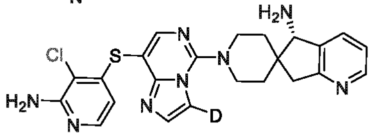
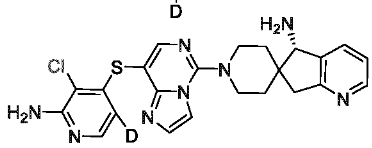
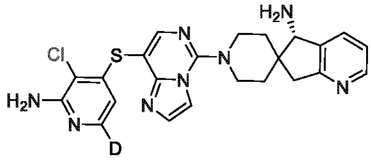
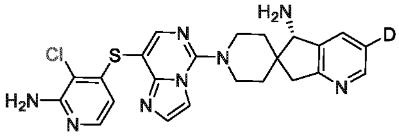
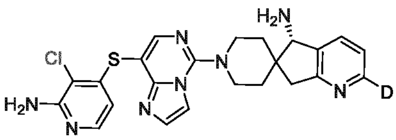
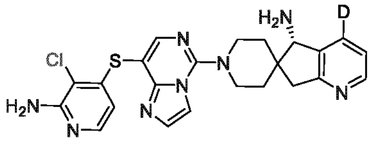
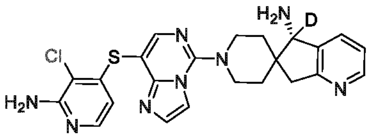
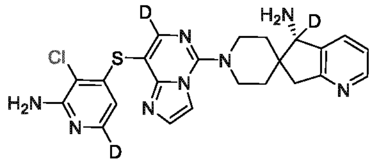
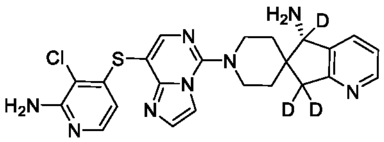

.

As a further preferred embodiment, the compound has a structure selected from the group consisting of:

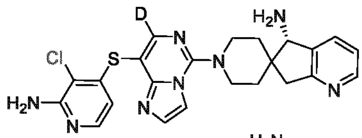
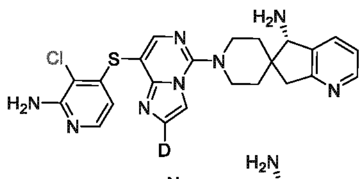
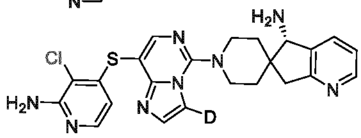
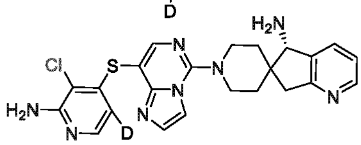
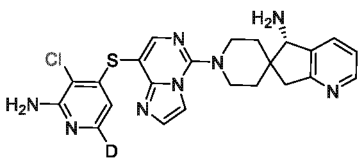
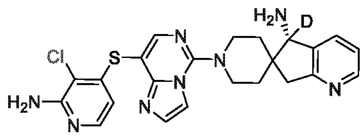

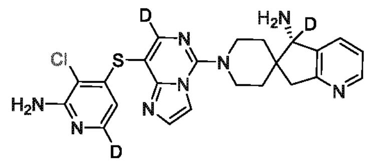

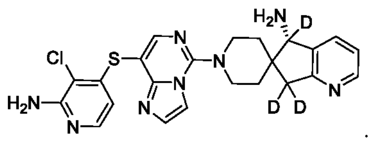

.

A second aspect of the present disclosure provides a method for preparing the compound of formula I according to the present disclosure, wherein the method comprises the following steps:

(i) reacting a compound of formula Ib with a compound of formula Ic by a nucleophilic substitution to obtain a compound of formula Id;
(ii) reacting the compound of formula Id with a compound of formula Ie by substitution to obtain a compound of formula If; and
(iii) deprotecting the compound of formula If with an acid to obtain the compound of formula I:

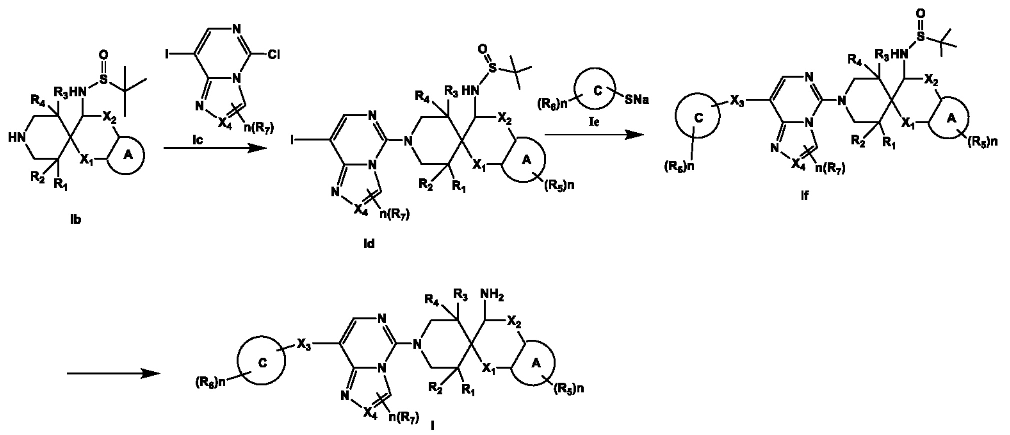

wherein, the ring A, $R_1$-$R_4$ and $X_1$ in the formula of IIc-6, IIc-7, IIc-8 and IIc are as described herein in any embodiment.

In the preparation method of the present disclosure, the corresponding isotope substituted starting reactantmay be used according to the isotope substituted-position of the compound of formula I. The isotope-substituted intermediate IIa of the intermediate of formula Ib is used as an example to illustrate the synthesis method. Other isotope-substituted intermediates can be used to synthesize the corresponding compounds with reference to the following methods.

In some aspects, the compound of formula Ib is a compound represented by the following formula IIc, which is prepared by using a method comprising the following steps:

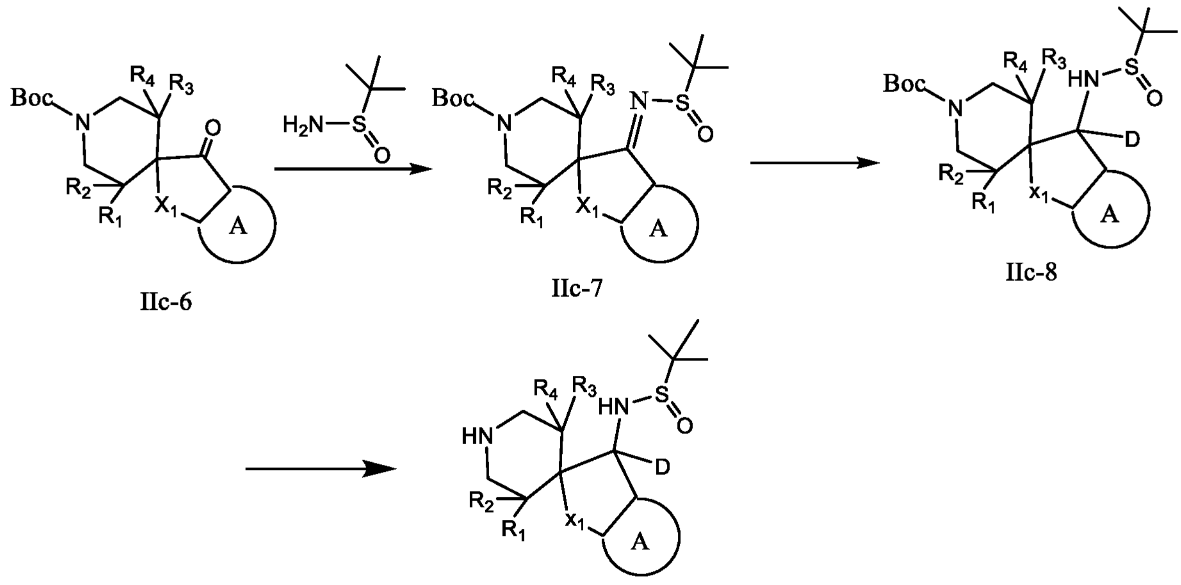

IIc-6 IIc-7 IIc-8

IIc

(1) A compound of formula IIc-6 is reacted with chiral tert-butyl sulfinamide in a solvent to obtain a compound of formula IIc-7; wherein the solvent is preferably an organotitanate compound, more preferably tetraisopropyl titanate.
(2) The compound of formula IIc-7 is reduced to a compound of formula IIc-8 by a deuterated reducing agent in a deuterated alcohol solvent; wherein the deuterated alcohol solvent is preferably deuterated methanol; and the deuterated reducing agent is preferably an alkali metal borodeuteride, more preferably potassium borodeuteride, sodium borodeuteride, most preferably sodium borodeuteride;
(3) The protective groups of the compound of formula IIc-8 is deprotected under the action of organic acids and haloalkanes to obtain the compound of formula IIc; wherein the organic acid is preferably haloalkyl acid, more preferably haloacetic acid, further preferably trifluoroacetic acid; the haloalkanes are preferably chlorinated alkanes, more preferably dichloromethane, carbon tetrachloride, further preferably dichloromethane.

In the method for preparing the compound of IIc, $X_1$, $R_1$-$R_4$ and ring A in each formula are as described herein in any embodiment.

In some aspects, the method of isotope substituting intermediates comprises the following steps:

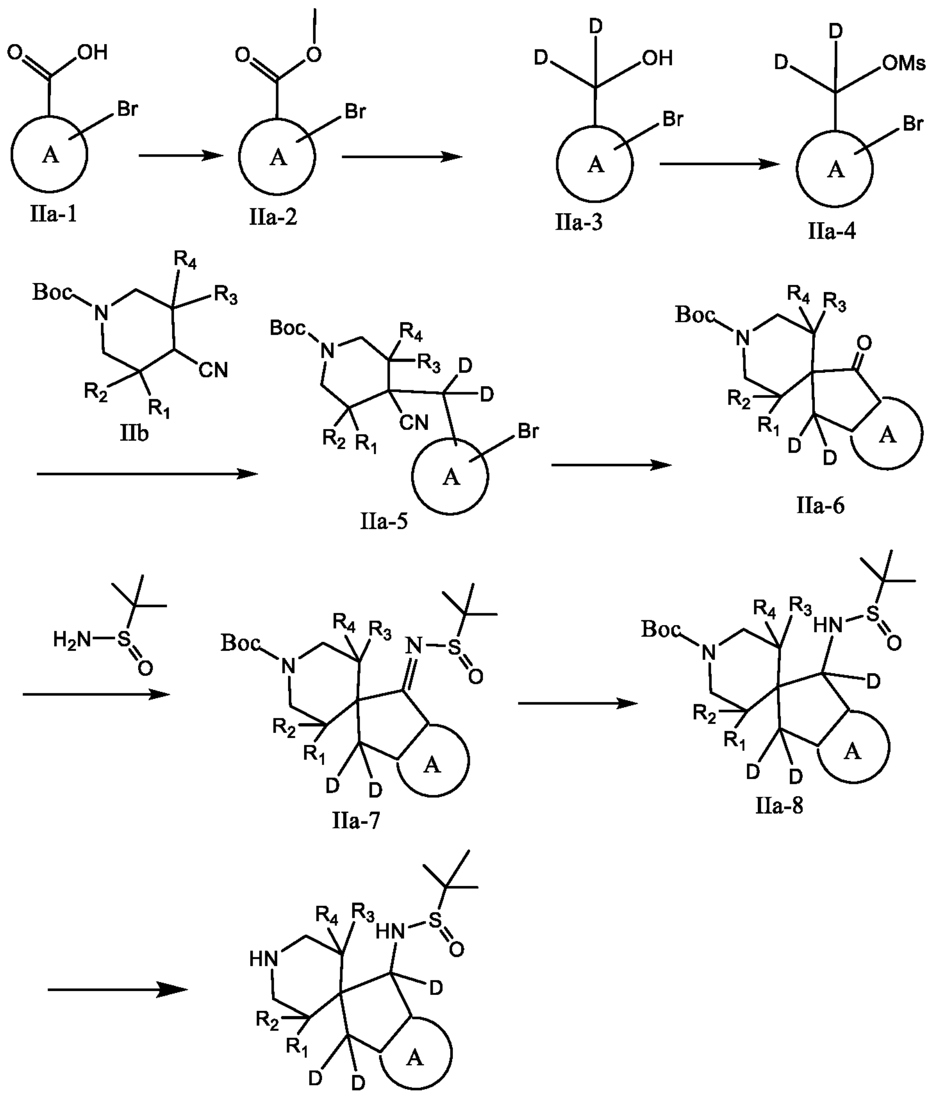

IIa-1 IIa-2 IIa-3 IIa-4 IIb IIa-5 IIa-6 IIa-7 IIa-8 IIa

(i) a compound of formula IIa-1 and alcohol solvents undergo esterification reaction at 60 to 100 °C for 6 to 20 hours under the catalysis of sulfoxide compounds to obtain a compound of IIa-2; wherein the alcohol solvent is preferably a fatty alcohol, more preferably methanol, ethanol, propanol, most preferably methanol; sulfoxide compounds preferably thionyl chloride, dimethyl sulfoxide, diphenyl sulfoxide, more preferably thionyl chloride. Preferably, the molar ratio of the compound of formula IIa-1 to sulfoxide compounds is preferably 1: 10-10: 1, preferably 1: 4-4: 1;

(ii) the compound of formula IIa-2 is reduced to a compound of formula IIa-3 by the deuterated reducing agent in a deuterated alcohol solvent; wherein the deuterated alcohol solvent is preferably deuterated alkyl alcohol, further preferably deuterated methanol, deuterated ethanol, most preferably deuterated methanol; and the deuterated reducing agent is preferably an alkali metal borodeuteride, more preferably potassium borodeuteride, sodium borodeuteride, most preferably sodium borodeuteride. Preferably, the mass ratio of the compound of formula IIa-2 to the deuterated reducing agent is preferably 1: 10-10: 1, more preferably 1: 3-2: 1;

(iii) the compound of formula IIa-3 is substituted with a methylsulfonyl group in the presence of an organic solvent and an organic base to obtain a compound of formula IIa-4; wherein the organic solvent is preferably a haloalkane, more preferably dichloromethane; and the organic base is preferably an alkyl amine, more preferably C1-3 alkyl amine, triethylamine. Preferably, the molar ratio of the compound of formula IIa-3 to methylsulfonyl group is 1: 10 to 11: 1, more preferably 1: 5-1: 1. Preferably, the compound of formula IIa-3 is reacted with methylsulfonyl chloride in the presence of an organic solvent and an organic base to obtain the compound of formula IIa-4;

(iv) in the presence of organic solvents and metal organic bases, the compound of formula IIa-4 is reacted with a compound of formula IIb to obtain a compound of formula IIa-5; wherein the metal organic base is preferably LDA; and the organic solvent is preferably an ether solvent, more preferably THF. Preferably, the molar ratio of the compound of formula IIa-4 to the compound of formula IIb is 1:1-10:1, preferably 1:2-2:1;

(v) the compound of formula IIa-5 is catalyzed by a palladium catalyst in the presence of an organic solvent and an organic base, and undergoes cyclization under heating to obtain a compound of formula IIa-6; wherein the palladium catalyst is preferably Pd (aMphos)$Cl_2$, the solvent is preferably DMA/$H_2O$, and the organic base is preferably triethylamine. Preferably, the molar ratio of the compound of formula IIa-5 to the palladium catalyst is 1 to 100, more preferably 5 to 20, most preferably 10;

(vi) the compound of formula IIa-6 is reacted with chiral tert-butyl sulfinamide in an organic solvent to obtain a compound of formula IIa-7; wherein the organic solvent is preferably an organotitanate compound, more preferably tetraisopropyl titanate, and the chiral tert-butylsulfinamide is preferably (R)-2-methylpropane-2-sulfinamide. Preferably, the molar ratio of the compound of formula IIa-6 to chiral tert-butyl sulfinamide is 1:10 to 2:1; more preferably 1: 5 to 2: 1;

(vii) the compound of formula IIa-7 is reduced to a compound of formula IIa-8 by a deuterated reducing agent in a deuterated alcohol solvent; wherein the deuterated alcohol solvent is preferably deuterated methanol; and the deuterated reducing agent is preferably an alkali metal borodeuteride, more preferably potassium borodeuteride, sodium borodeuteride, most preferably sodium borodeuteride. Preferably, the molar ratio of the compound of formula IIa-7 to the deuterated reducing agent is 1:10 to 2:1; further preferably 1: 5 to 2: 1;

(viii) the protective groups of the compound of formula IIa-7 is deprotected under the action of organic acids and haloalkanes to obtain the compound of formula IIa; wherein the organic acid is preferably haloalkyl acid, more preferably haloacetic acid, further preferably trifluoroacetic acid, the haloalkane is preferably a chlorinated alkane, more preferably dichloromethane, carbon tetrachloride, further preferably dichloromethane.

In some aspects, the method of producing the isotope substituted intermediate IIa may also comprise only three steps of the above (vi), (vii) and (viii).

The definitions of rings and substituents such as ring A, $R_1$-$R_4$ involved in the reaction scheme for the above intermediates are as described in any embodiment herein.

A third aspect of the present disclosure provides use of an isotope substituted compound represented by formula I of the present disclosure in the following methods:

(a) preparing drugs for the prevention or treatment of diseases or disorders associated with abnormal activity/level of SHP2;
(b) preparing drugs for the prevention or treatment of SHP2-mediated diseases or disorders;
(c) preparing inhibitor drugs that inhibit SHP2 activity/level;
(d) non-therapeutically inhibiting SHP2 activity/level in vitro;
(e) non-therapeutically inhibiting tumor cell proliferation in vitro; or
(f) treating diseases or conditions associated with abnormal SHP2 activity/level.

The "SHP2-mediated disease or disorder" refers to a disease or disorder associated with abnormal SHP2 activity/level, which may be abnormal increase or decrease in SHP2 activity/level caused by abnormal activation or destruction of SHP2 in the subject’s body. In certain embodiments, the SHP2-mediated disease or disorder is associated with an abnormal increase in SHP2 activity/level. In certain embodiments, the SHP2-mediated disease or disorder is cancer.

In a preferred embodiment, the disease is SHP2-mediated cancer; preferably Noonan syndrome, Leopard syndrome, adolescent myelomonocytic leukemia, neuroblastoma, melanoma, acute myeloid leukemia, breast cancer, esophageal cancer, lung cancer, colon cancer, head cancer, neuroblastoma, squamous cell carcinoma of the head and neck, gastric cancer, anaplastic large cell lymphoma, glioblastoma, hepatocellular carcinoma (HCC), acute lymphoblastic leukemia, adrenal cortex carcinoma, anal cancer, appendix cancer, astrocytomas, atypical malformations/tumoroids, basal cell carcinoma, cholangiocarcinoma, bladder cancer, bone cancer (osteosarcoma and malignant fibrous histiocytoma), brainstem glioma, brain tumor, brain and spinal cord tumor, bronchial tumor, Burkitt lymphoma, cervical cancer, chronic lymphocytic leukemia, chronic myelogenous leukemia, colorectal cancer, craniopharyngioma, embryonic tumor, endometrial cancer, epithelial cell tumors, ependymomas, Ewing sarcoma family tumors, eye cancer, retinoblastoma, gallbladder carcinoma, gastrointestinal carcinoid, gastrointestinal stromal tumors (GIST), gastrointestinal stromal cell tumors, germ cell tumors, gliomas, hair cell leukemia, head and neck cancer, Hodgkin lymphoma, hypopharyngeal cancer, islet cell tumor (endocrine pancreas), Kapozi sarcoma, kidney cancer, Langerhans cell histiocytosis, laryngeal cancer, leukemia, hair cell leukemia, liver cancer, non-small cell lung cancer, small cell lung cancer, lymphoma, medulloblastoma, medullary epithelioma, mesothelioma, oral cancer, multiple myeloma, nasopharyngeal cancer, neuroblastoma, non-Hodgkin lymphoma, oropharyngeal cancer, osteosarcoma, malignant bone fibrous histiocytoma, ovarian cancer, ovarian epithelial carcinoma, ovarian germ cell tumor, ovarian low malignant potential tumor, pancreatic cancer, papillomatosis, parathyroid carcinoma, penile cancer, pharyngeal cancer, pineal intermediate differentiation tumor, osteoblastoma and supratentorial primitive neuroectodermal tumor, pituitary tumor, plasma cell tumor/multiple myeloma,

pleural pneumocytoma, primary central nervous system lymphoma, prostate cancer, rectal cancer, kidney cell (kidney) cancer, retinoblastoma, rhabdomyosarcoma, salivary adenocarcinoma, sarcoma, Ewing sarcoma family tumors, sarcoma, Kaposi disease, Sezary syndrome, skin cancer, small intestinal carcinoma, soft tissue sarcoma, squamous cell carcinoma, supratentorial primitive neuroectodermal tumor, T-cell lymphoma, testicular cancer, laryngeal cancer, thymoma and thymus cancer, thyroid cancer, urethral cancer, uterine cancer, uterine sarcoma, vaginal cancer, vulvar cancer, Waldenstrom macroglobulinemia and Wilms tumors.

A fourth aspect of the present disclosure provides a pharmaceutical composition comprising:

(i) an effective amount of the isotope-substituted compound represented by formula I, or a pharmaceutically acceptable salt, an enantiomer, a diastereomer, a tautomer, a solvate, a polymorph, a prodrug or a metabolite thereof; and
(ii) pharmaceutically acceptable carriers.

A fifth aspect of the present disclosure provides a method of inhibiting SHP2 activity comprising the following steps: administering an effective amount of a compound of formula I according to the present disclosure or a pharmaceutically acceptable salt thereof to a subject in need thereof, or administering an effective amount of a pharmaceutical composition according to the present disclosure to a subject in need thereof.

A sixth aspect of the present disclosure provides a method of preventing or treating a disease or disorder associated with abnormal activity of SHP2, which comprises administering an effective amount of the isotope-substituted compound represented by formula I according to the present disclosure or a pharmaceutically acceptable salt thereof, or an enantiomer, a diastereomer, a tautomer, a solvate, a polymorph, a prodrug or a metabolite thereof to a subject in need thereof, or administering an effective amount of the pharmaceutical compositionaccording to the present disclosure to a subject in need thereof.

A seventh aspect of the present disclosure provides a pharmaceutical composition further comprising other therapeutic agents. Said other therapeutic agents include anticancer drugs, for example, abarelix, aldesleukin, alemtuzumab, alitretinoin, allopurinol, altretamine, amifostine, anastrozole, arsenic trioxide, asparaginase, azacitidine, BCG Live, bevacuzimab, fluorouracil, bexarotene, bleomycin, bortezomib, busulfan, calusterone, capecitabine, camptothecin, carboplatin, carmustine, celecoxib, cetuximab, chlorambucil, cladribine, clofarabine, cyclophosphamide, cytarabine, actinomycin D, darbepoetin alfa, daunomycin, denileukin, dexrazoxane, docetaxel, doxorubicin (neutral), hydrochloric acid doxorubicin, dromostanolone propionate, epirubicin, epoetin alfa, erlotinib, estramustine, etoposide phosphate, etoposide, exemestane, filgrastim, floxuridine fludarabine, fulvestrant, gefitinib, gemcitabine, gemtuzumab, goserelin acetate, histrelin acetate, hydroxyurea, ibritumomab, idarubicin, ifosfamide, imatinib mesylate, interferon $\alpha$-2a, interferon $\alpha$-2b, irinotecan, lenalidomide, letrozole, leucovorin, leuprolide acetate, levamisole, lomustine, megestrol acetate, melphalan, mercaptopurine, 6-MP, mesna, methotrexate, methoxsalen, mitomycin C, mitotane, mitoxantrone, nandrolone, nelarabine, nofetumomab, oprelvekin, oxaliplatin, paclitaxel, palifermin, pamidronate, pegademase, pegaspargase, pegfilgrastim, pemetrexed disodium, pentostatin, pipobroman, plicamycin, porfimer sodium, procarbazine, quinacrine, rasburicase, rituximab, sargramostim, sorafenib, streptozocin, sunitinib maleate, talc, tamoxifen, temozolomide, teniposide, VM-26, testolactone, thioguanine, 6-TG, thiotepa, topotecan, toremifene, tositumomab, rastuzumab, tretinoin, ATRA, uracil mustard, valrubicin, vinblastine, vincristine, vinorelbine, zoledronate or zoledronic acid.

An eighth aspect of the present disclosure provides a method of preparing a pharmaceutical composition, which comprises mixing an isotope substituted compound represented by formula I or a pharmaceutically acceptable salt thereof, or an enantiomer, a diastereomer, a tautomer, a solvate, a polymorph, a prodrug or a metabolite thereof with a pharmaceutically acceptable carrier.

It should be understood that, within the scope of the present disclosure, the above technical features of the present disclosure and the technical features specifically described below (e.g., in examples) may be combined with each other to constitute a new or preferred technical solution. Due to space limitations, they are not repeated here.

**Embodiment**

After long-term and in-depth research, the present inventors have prepared a class of novel allosteric inhibitor compounds having formula I, which can be combined with non-catalytic regions of SHP2 and keep a self-inhibiting state with very weak activity of SHP2 "locked", thereby achieving the purpose of inhibiting its activity. The compound of the present disclosure exhibits good biological activity and druggability, has good drug development prospects. It inhibits SHP2 with quite excellent inhibitory activity even at very low concentrations (may be as low as $\leq$100nM), and thus can be used to treat SHP2-related diseases or disorders, such as tumors. Further, in the present disclosure, hydrogen atoms at one or more positions of the compound of formula I are selected to be deuterated. The resulting deuterated compound has significantly reduced metabolism rate, significantly improved plasma half-life, significantly improved blood concentration, thereby significantly improving the efficacy of the drug and reducing the toxic side effects of the drug. Based on the above findings, the inventors completed the present disclosure.

**Terms**

Unless otherwise defined, all scientific and technical terms herein have the same meaning as those generally understood by those skilled in the art to which the claims relate. Unless otherwise indicated, all patents, patent applications, and disclosure materials cited in the text of the present disclosure are incorporated herein by reference.

It should be understood that the foregoing brief description and the details below are exemplary and for interpretation only, without any restrictions on the subject matter of the present disclosure. In the present disclosure, unless otherwise specified, the plural is also included when the singular is used. It must be noted that, unless otherwise clearly stated herein, the singular form used in this specification and claims includes the plural form of the term being referred to. It should also be noted that, unless otherwise indicated, the term "or" means "and/or". Further, the term "comprise" and other forms used, such as "comprising", "containing" and "having", are not restrictive, which may be open, semi-closed and closed. In other words, the term also includes the meaning of "substantially consist of ... ", or "consist of... ".

Definitions of standard chemical terms can be found in references including Carey and Sundberg, "ADVANCED ORGANIC CHEMISTRY 4TH ED.", Vols. A (2000) and B (2001), Plenum Press, New York. Unless otherwise indicated, conventional methods in the art are employed, such as mass spectrometry, NMR, IR and UV/VIS spectroscopy and pharmacological methods. Unless specific definitions are proposed, the terms used herein in descriptions related to the analytical chemistry, organic synthetic chemistry, and pharmaceutical and medicinal chemistry are known in the art. Standard techniques can be used in chemical synthesis, chemical analysis, drug preparation, formulation and delivery, as well as in the treatment of patients. For example, the reaction and purification may be implemented according to the instructions for the use of the kit provided by the manufacturer, or in accordance with the well-known manner in the art or the description of the present disclosure. Generally, according to a plurality of descriptions in general or detailed literatures cited and discussed in this specification, the above techniques and methods are implemented in accordance with conventional methods well known in the art. In the present specification, those skilled in the art may select groups and substituents thereof to provide a stable structural portion and a compound.

When describing a substituent by conventional chemical formula written from left to right, the substituent likewise comprises a chemically equivalent substituent obtained when writing a structural formula from right to left. For example, $-CH_2O-$ is equivalent to $-OCH_2-$.

The section headings used herein are for the purpose of organizing the article only and should not be construed as restricting the subject. All literature or portions of literature cited in the present disclosure, including but not limited to patents, patent applications, articles, books, operating manuals and papers, are incorporated herein by reference.

Some of the chemical groups defined herein are preceded by simplified symbols to represent the total number of carbon atoms present in that group. For example, C1-C6 alkyl refers to an alkyl group having a total of 1 to 6 carbon atoms as defined below. The total number of carbon atoms in the simplified symbol does not include carbon that may be present in the substituent of the group.

In addition to the foregoing, when used in the specification and claims of the present disclosure, unless otherwise specifically indicated, the following terms have the meanings shown below.

In the present disclosure, the term "halogen" refers to fluorine, chlorine, bromine or iodine.

"Deuterium" can be denoted by "D".
"Hydroxyl" means -OH group.
"Hydroxyalkyl" refers to an alkyl group as defined hereinafter substituted with a hydroxyl group (-OH).
"Carbonyl" refers to -C(=O)-.
"Nitro" refers to-$NO_2$.
"Cyano" refers to -CN.
"Amino" refers to $-NH_2$.
"Substituted amino" refers to amino substituted with one or two alkyl groups, alkyl carbonyl, aralkyl, heteroaryl alkyl as defined below, e.g., monoalkyl amino, dialkyl amino, alkanoyl amino, aralkyl amino, heteroaryl alkylamino.
"Carboxyl" refers to -COOH.

In the present disclosure, as a group or a part of other groups (e.g., in alkyl groups substituted with halogens (e.g., fluorine, chlorine, bromine or iodine)), the term "alkyl" refers to a fully saturated linear or branched hydrocarbon chain group, which is composed only of carbon atoms and hydrogen atoms, having, for example, 1 to 12 (preferably 1 to 8, more preferably 1 to 6) carbon atoms, and is connected to the rest of the molecule by a single bond. For example, it includes but not limited to methyl, ethyl, n-propyl, isopropyl, n-butyl, isobutyl, sec-butyl, tert-Butyl, n-pentyl, 2-methylbutyl, 2,2-dimethylpropyl, n-hexyl, heptyl, 2-methylhexyl, 3-methylhexyl, octyl, nonyl and decyl, etc. For purposes of the present disclosure, the term "alkyl" refers to an alkyl group containing 1 to 8 carbon atoms.

In the present disclosure, as a group or a part of other groups, the term "alkenyl" refers to a linear or branched hydrocarbon chain group which is composed only of carbon atoms and hydrogen atoms, containing at least one double

bond, having, for example, 2 to 20 (preferably 2 to 10, more preferably 2 to 6) carbon atoms and is connected to the rest of the molecule by a single bond. For example, it includes but not limited to vinyl, propenyl, allyl, 1-butenyl, 2-butenyl, 1-pentenyl, penta-1,4-dienyl and the like.

In the present disclosure, as a group or a part of other groups, the term "cyclic hydrocarbyl" refers to a stable non-aromatic monocyclic or polycyclic hydrocarbyl (for example, alkyl, alkenyl or alkynyl) composed only of carbon and hydrogen atoms, which may include a fused ring system, a bridge ring system or a spiral ring system, having 3 to 15 carbon atoms, preferably having 3 to 10 carbon atoms, more preferably having 3 to 8 carbon atoms, such as 3, 4, 5, 6, 7 or 8 carbon atoms. It is saturated or unsaturated and connected to the rest of the molecule by a single bond via any suitable carbon atom. Unless otherwise specified in the present specification, the carbon atoms in the cyclic hydrocarbyl may be optionally oxidized. Examples of cyclic hydrocarbyl include, but are not limited to, cyclopropyl, cyclobutyl, cyclopentyl, cyclopentenyl, cyclohexyl, cyclohexenyl, cyclohexadienyl, cycloheptyl, cyclooctyl, 1H-indenyl, 2,3-indanyl, 1,2,3,4-tetrahydro-naphthyl, 5,6,7,8-tetrahydro-naphthyl, 8,9-dihydro-7H-benzocycloheptene-6-yl, 6,7,8,9-tetrahydro-5H-benzocycloheptenyl, 5,6,7,8,9,10-hexahydro-benzocyclooctenyl, fluorenyl, dicyclo [2.2.1] heptyl, 7,7-dimethyl-dicyclo[2.2.1] heptyl, dicyclo [2.2.1] heptenyl, dicyclo [2.2.2] octyl, dicyclo [3.1.1] heptyl, dicyclo [3.2.1] octyl, dicyclic [2.2.2] octenyl, dicyclo [3.2.1] octenyl, adamantyl, octahydro-4,7-methylene-1H-indenyl and octahydro-2,5-methylene-cyclopentadieno and the like.

In the present disclosure, as a group or a part of other group, the term "heterocyclic group" refers to a stable 3 to 20 membered non-aromatic cyclic group composed of 2 to 14 carbon atoms (e.g., 2, 3, 4, 5, 6, 7, 8, 9, 10, 11, 12, 13 or 14 carbon atoms) and 1 to 6 heteroatoms selected from nitrogen, phosphorus, oxygen and sulfur. Unless otherwise specified in this specification, the heterocyclic group may be a ring system of a single ring, a double ring, three rings or more rings, which may include a fused ring system, a bridged ring system or a spiral ring system; wherein the nitrogen, carbon or sulfur atoms in the heterocyclic group can be optionally oxidized; wherein nitrogen atoms may be optionally quaternarized; and the heterocyclic group may be partially or fully saturated. Heterocyclyl can be connected to the rest of the molecule via carbon atoms or heteroatoms by a single bond. In a heterocyclic group comprising a fused ring, one or more rings may be an aryl group or heteroaryl as defined below, provided that the connection point with the rest of the molecule is a non-aromatic ring atom. For the purpose of the present disclosure, the heterocyclic group is preferably a stable 4- to 11-membered non-aromatic monocyclic, bicyclic, bridged or spiral ring group comprising 1 to 3 heteroatoms selected from nitrogen, oxygen and sulfur, preferably a stable 4- to 8-membered non-aromatic monocyclic, bicyclic, bridged or spiral ring group comprising 1 to 3 heteroatoms selected from nitrogen, oxygen and sulfur. Examples of heterocyclyl include, but are not limited to: pyrrolidyl, morpholinyl, piperazinyl, homopiperazinyl, piperidinyl, thiomorpholinyl, 2,7-diaza-spiro[3.5]nonane-7-yl, 2-oxa-6-aza-spiro[3.3]heptane-6-yl, 2,5-diaza-biscyclo[2.2.1]heptane-2-yl, azetidinyl, pyranyl, tetrahydropyranyl, thiopyranyl, tetrahydrofuranyl, oxyazinyl, dioxolanyl, tetrahydroisoquinolinyl, decahydroisoquinolinyl, imidazolinyl, imidazolidinyl, quinolizinyl, thiazolidinyl, isothiazodinyl, isoxazolidinyl, indolinyl, octahydroindolyl, octahydroisoindolyl, pyrrolidinyl, pyrazolidinyl, phthalimido, etc.

In the present disclosure, as a group or a part of other group, the term "aryl" refers to a conjugated hydrocarbon ring system group having 6 to 18 carbon atoms (preferably having 6 to 10 carbon atoms, e.g., 6, 7, 8, 9 or 10 carbon atoms). For the purpose of the present disclosure, the aryl group may be a ring system of monocyclic, bicyclic, three or more rings and may also be fused with the above-defined cycloalkyl or heterocyclyl, provided that the aryl group is connected to the rest of the molecule via atoms on the aromatic ring by a single bond. Examples of aryl groups include, but are not limited to, phenyl, naphthyl, anthryl, phenanthryl, fluorenyl, 2,3-dihydro-1H-isoindolyl, 2-benzoxazolonyl, 2H-1,4-benzoxazin-3(4H)-one-7-yl and the like.

In the present disclosure, the term "aryl alkyl" refers to an alkyl group as defined above which is substituted by an aryl group as defined above.

In the present disclosure, as a group or a part of other group, the term "heteroaryl" refers to a 5 to 16 membered conjugated ring group having 1 to 15 carbon atoms (preferably having 1 to 10 carbon atoms, e.g., 1, 2, 3, 4, 5, 6, 7, 8, 9 or 10 carbon atoms) and 1 to 6 heteroatoms selected from nitrogen, oxygen and sulfur. Unless otherwise specified in the present disclosure, heteroaryls may be a ring system of monocyclic, bicyclic, three or more rings, and may also be fused with the above-defined cycloalkyl or heterocyclyl, provided that the heteroaryl is connected to the rest of the molecule via atoms on the aromatic ring by a single bond. Nitrogen, carbon or sulfur atoms in heteroaryl can be optionally oxidized and nitrogen atoms may be optionally quaternarized. For the purpose of the present disclosure, the heteroarylis preferably a stable 5- to 12-membered aromatic group comprising 1-5 heteroatoms selected from nitrogen, oxygen and sulfur, more preferably a stable 5- to 10-membered aromatic group comprising 1 to 4 heteroatoms selected from nitrogen, oxygen and sulfur or a 5 to 6-membered aromatic group comprising 1 to 3 heteroatoms selected from nitrogen, oxygen and sulfur. Examples of heteroaryl include, but are not limited to, thienyl, imidazolyl, pyrazoyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furanyl, pyrrolyl, triazolyl, tetrazolyl, triazinyl, indolizinyl, isoindolyl, indazolyl, isoindazolyl, purinyl, quinolyl, isoquinolyl, naphthyridinyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, phenylthio, indolizinyl, phenanthrolinyl, isooxazolyl, phenoxazinyl, phenothiazinyl, 4,5,6,7-tetrahydrobenzo[b]thiophene, naphthopyridyl, [1,2,4]triazolo[4,3-b]pyridazine, [1,2,4]triazolo[4,3-a]pyrazine, [1,2,4]triazolo[4,3-c]pyrimidine, [1,2,4]triazolo[4,3-a]pyridine, imidazo[1,2-a]pyridine, imidazo[1,2-b]pyridazine, imidazo[1,2-a]pyrazine, etc.

In the present disclosure, the term "heteroaryl alkyl" refers to an alkyl group as defined above substituted with a heteroaryl as defined above.

In the present disclosure, "optional" or "optionally" means that the event or situation subsequently described may or may not occur, and the expression includes both the occurrence and non-occurrence of the event or situation. For example, "optionally substituted aryl" means that the aryl group is substituted or unsubstituted, and the expression includes both the substituted aryl group and the unsubstituted aryl group. The "optional" substituents as described in the claims and specifications of the present disclosure are selected from alkyl, alkenyl, alkynyl, halogen, haloalkyl, haloalkenyl, haloalkynyl, cyano, nitro, optionally substituted aryl, optionally substituted heteroaryl, optionally substituted cyclic hydrocarbyl, optionally substituted heterocyclic group.

"SHP2" refers to "Src Homolgy-2 phosphatase", also called SH-PTP2, SH-PT3, Syp, PTP1D, PTP2C, SAP-2 or PTPN11.

As used herein, the terms "part", "structural part", "chemical part", "group", "chemical group" refer to a particular fragment or functional group in a molecule. The chemical part is often considered as a chemical entity embedded or attached to a molecule.

"Stereoisomer" refers to a compound consisting of the same atoms bonded by the same bond, but with a different three-dimensional structure. The present disclosure covers a variety of stereoisomers and mixtures thereof.

When the compounds of the present disclosure contain ethylenic double bonds, unless otherwise indicated, the compounds of the present disclosure are intended to comprise E- and Z- geometric isomers.

"Tautomer" refers to the isomer formed by the transfer of protons from one atom of a molecule to another atom of the same molecule. All tautomeric forms of compounds of the present disclosure are also included within the scope of the present disclosure.

Compounds of the present disclosure or pharmaceutically acceptable salts thereof may contain one or more chiral carbon atoms, and may therefore produce enantiomers, diastereomers and other stereoisomers. Each chiral carbon atom may be defined as (R)- or (S)-based on stereochemistry. The present disclosure is intended to include all possible isomers, as well as racemates and optical pure forms thereof. Racemates, diastereomers or enantiomers may be selected as raw materials or intermediates for the preparation of compounds of the present disclosure. Optically active isomers can be prepared using chiral synthons or chiral reagents, or separated using conventional techniques, such as crystallization and chiral chromatography.

Conventional techniques for preparing/separating individual isomers include chiral synthesis from suitable optically pure precursors, or using, for example, chiral high performance liquid chromatography to separate racemates (or racemates of salts or derivatives), as may be seen in, for example, Gerald Gübitz and Martin G. Schmid (Eds.), Chiral Separations, Methods and Protocols, Methods in Molecular Biology, Vol. 243, 2004; A.M. Stalcup, Chiral Separations, Annu. Rev. Anal. Chem. 3:341-63, 2010; Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res. 1990, 23, 128.

The isotope substitution of the present disclosure refers to one or more ring carbon atoms in one or more rings of ring A, ring B, ring C, ring D, ring E and ring F in the compound represented by formula I are substituted with $^{13}C$, and/or hydrogen atoms on one or more ring atoms of one or more rings of ring A, ring B, ring C, ring D, ring E and ring F are substituted with deuterium.

It is particularly preferred in the present disclosure that hydrogen atoms at one or more positions selected from the following positionsare deuterated: hydrogen atoms at any positions of the ring atoms other than the heterocyclic atom in the ring A; and/or, hydrogen atoms on the ring atoms substituted with amino in the ring B; and/or, hydrogen atoms at any positions of the ring atoms other than the heterocyclic atom in the ring C; and/or, hydrogen atoms at 7-position of the ring atom in the ring E; and/or, hydrogen atoms at 2- and 3-positions of the ring atoms in the ring F. In the compounds of formula I of the present disclosure, the number of deuterium is typically 1-4, preferably 1-2.

Isotope variants of the compounds of the present disclosure or pharmaceutically acceptable salts thereof may be prepared by conventional techniques using appropriate isotopic variants of suitable agents.

In the present disclosure, the term "pharmaceutically acceptable salt" includes a pharmaceutically acceptable acid addition salt and a pharmaceutically acceptable base addition salt.

"Pharmaceutically acceptable acid addition salt" refers to a salt formed from an inorganic or organic acid that is capable of maintaining the bioavailability of a free base without other side effects. Inorganic acids include, but are not limited to, hydrochloride, hydrobromide, sulfate, nitrate, phosphate, etc. Organic acids include, but are not limited to, formate, acetate, 2,2-dichloroacetate, trifluoroacetate, propionate, caproate, caprylate, decanoate, undecylenate, glycolate, glucorate, lactate, sebacate, adipate, glutarate, malonate, oxalate, maleate, succinate, fumarate, tartarate, citrate, palmitate, stearate, oleate, cinnamate, laurate, malate, glutamic acid salt, pyroglutamate, aspartate, benzoate, methanesulfonate, benzenesulfonate, p-toluenesulfonate, alginate, ascorbate, salicylate, 4-aminosalicylate, naphthalene disulfonate, etc. These salts can be prepared by methods known in the technical field.

"Pharmaceutically acceptable base addition salt" refers to a salt formed from an inorganic or organic base that is capable of maintaining the bioavailability of a free acid without other side effects. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts and magnesium salts. Salts derived from organic bases include, but are not limited to, the salts derived from the following amines: primary amines, secondary and tertiary amines, substituted amines including natural substituted amines, cyclic amines and alkaline ion exchange resins such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methyl glucosamine, theobromine, purine, piperazine, piperidine, N-ethylpiperidine, polyamine resin, etc. Preferred organic bases include isopropylamine, diethylamine, ethanolamine, trimethylamine, dicyclohexylamine, choline and caffeine. These salts can be prepared by methods known in the technical field.

In the present disclosure, "pharmaceutical compositions" refers to a formulation of a compound of the present disclosure and commonly acceptable medium for delivering a biologically active compound to a mammal (e.g., a human) in the art. This medium comprises a pharmaceutically acceptable carrier. The object of the pharmaceutical composition is to promote the administration to an organism, facilitate the absorption of the active ingredient and thus exert biological activity.

As used herein, the term "pharmaceutically acceptable" refers to a substance (e.g., a carrier or diluent) that does not affect the biological activity or properties of the compounds of the present disclosure, and is relatively nontoxic, i.e., the substance may be administered to an individual without causing adverse biological reactions or interacting with any component contained in the composition in an undesirable manner.

In the present disclosure, "pharmaceutically acceptable excipients" include, but are not limited to, any adjuvants, carriers, excipients, flow aids, sweeteners, diluents, preservatives, dyes/colorants, flavoring agents, surfactants, wetting agents, dispersants, suspensions, stabilizers, isotonic agents, solvents or emulsifiers licensed by the relevant government administration as acceptable for human or livestock use.

The "tumor" of the present disclosure includes, but is not limited to, diseases such as Noonan syndrome, leopard syndrome, juvenile myelomonocytic leukemia, neuroblastoma, sarcoma, melanoma, articular chondroma, cholangioma, leukemia, breast cancer, gastrointestinal stromal tumor, histiocytic lymphoma, non-small cell lung cancer, small cell lung cancer, esophageal cancer, pancreatic cancer, lung squamous cell carcinoma, lung adenocarcinoma, breast cancer, prostate cancer, liver cancer, skin cancer, epithelial cell carcinoma, cervical cancer, ovarian cancer, intestinal cancer, nasopharyngeal cancer, brain cancer, bone cancer, kidney cancer, oral cancer/head cancer, neuroblastoma, squamous cell carcinoma of the head and neck, anaplastic large cell lymphoma or glioblastoma, etc.

As used herein, the terms "preventive", "prevent" and "prevention" include reducing the likelihood of the occurrence or exacerbation of the disease or disorder in a patient.

As used herein, the term "therapeutic" and other similar synonyms include the following meanings:

(i) preventing the appearance of a disease or disorder in mammals, in particular when such mammals are susceptible to the disease or disorder but have not yet been diagnosed with the disease or disorder;
(ii) inhibiting the disease or disorder, i.e. curbing its development;
(iii) alleviating the disease or disorder, i.e., to subside the state of the disease or disorder; or
(iv) alleviating the symptoms caused by the disease or disorder.

As used herein, the term "effective amount", "therapeutically effective amount" or "pharmaceutically effective amount" refers to the amount of at least one agent or compound sufficient to alleviate one or more symptoms of the disease or disorder to be treated to some extent after administration. The result may be a reduction and/or remission of signs, symptoms or causes, or any other desired change in a biological system. For example, the "effective amount" for treatment is the amount of a composition comprising the compound disclosed herein required to provide a clinically significant relief effect of the disorder. Techniques such as dose-escalation tests may be used to determine effective amounts suitable for any individual case.

As used herein, the terms "taking", "application", "administration" and the like refer to a method capable of delivering a compound or composition to a desired site for biological action. These methods include, but are not limited to, oral route, transduodenal route, parenteral injection (including intravenous, subcutaneous, intraperitoneal, intramuscular, intraarterial injection or infusion), topical administration and transrectaladministration. Those skilled in the art are familiar with the application techniques available for compounds and methods described herein, e.g., those discussed in Goodman and Gilman, The Pharmacological Basis of Therapeutics, current ed.; Pergamon; and Remington's, Pharmaceutical Sciences (current edition), Mack Publishing Co., Easton, Pa. In a preferred embodiment, the compounds and compositions discussed herein are administered orally.

As used herein, the terms "combination of drugs", "drug combination", "concomitant drugs", "administration of other treatments", "administration of other therapeutic agents" and the like refer to a drug therapy obtained by mixing or combining more than one active ingredients, which include fixed combinations and irregular combinations of active ingredients. The term "fixed combination" refers to simultaneous administration of at least one compound described herein and at least one synergistic agent to a patient in a form of a single entity or a single dosage. The term "irregular combination" refers to simultaneous administration to the patient in the form of a separate entity, combined administration or sequential administration at variable intervals of at least one compound described herein and at least one synergistic formulation. These are also applied to cocktail therapies, such as administration of three or more active ingredients.

Those skilled in the art should also understand that, in the method described below, functional groups of the intermediate compound may need to be protected by appropriate protective groups. Such functional groups include hydroxyl, amino, mercapto and carboxylic acid. Suitable hydroxyl protective groups include trialkyl silyl or diaryl alkyl silyl (e.g., tert-butyl dimethylsilyl, tert-butyldiphenyl silyl or trimethylsilyl), tetrahydropyranyl, benzyl and the like. Suitable protective groups for amino, amidino and guanidyl include tert-butoxycarbonyl, benzyloxycarbonyl and the like. Suitable protective groups for mercapto include -C(O)-R" (wherein R" is alkyl, aryl or aralkyl), p-methoxybenzyl, triphenylmethyl and the like. Suitable protective groups for carboxyl include alkyl, aryl or aralkyl esters.

The protective groups may be introduced and removed according to standard techniques known by those skilled in the art and described herein. The use of protective groups is described in detail in Greene, T. W. and P. G. M. Wuts, Protective Groups in Organi Synthesis, (1999), 4th Ed., Wiley. The protective group may also be a polymer resin.

**Preparation of compounds of formula I**

The compound of formula I provided by the present disclosure may be prepared by the following method: reacting a compound of formula Ib with a compound of formula Ic by a nucleophilic substitution to obtain a compound of formula Id; reacting the compound of formula Id with a compound of formula Ie by substitution to obtain a compound of formula If; and deprotecting the compound of formula If with an acid to obtain the compound of formula I:

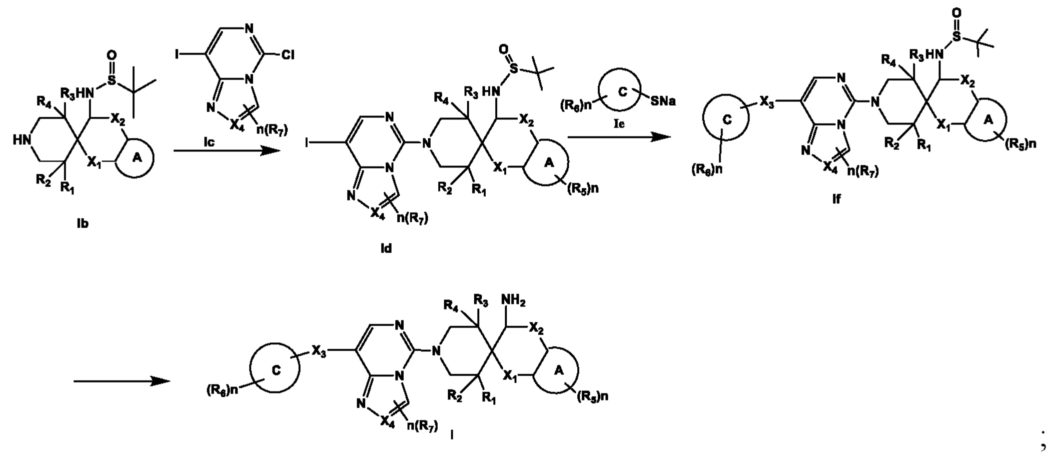

;

wherein the definition of each group is as defined above. In the above preparation method, the corresponding isotope substituted starting reactants may be used according to the isotope substituted position of the compound of formula I.

**Pharmacology and uses**

Src Hommolgy-2 phosphatase (SHP2) is a protein tyrosine phosphatase encoded by the PTPN11 gene that promotes a variety of cellular functions, including proliferation, differentiation, cell cycle maintenance, and migration. SHP2 is involved in the signaling of Ras-mitogen-activated protein kinase, JAK-STAT or phosphoinositol 3-kinase-AKT pathways. SHP2 mediates activation of receptor tyrosine kinases such as ErbB 1, ErbB2 and c-Met's Erk1 and Erk2 MAP kinases.

SHP2 has two N-terminal Src homolgy 2 domains (N-SH2 and C-SH2), a catalytic domain (PTP) and C-terminal tail. The two SH2 domains control subcellular localization and functional regulation of SHP2. The molecule exists as an inactive conformation, inhibiting its own activity through a binding network involving residues from the N-SH2 and PTP domains. In response to growth factor stimulation, SHP2 binds to specific tyrosine-phosphorylation sites such as Gab

1 and Gab2 on the docking protein via its SH2 domain. This causes conformational changes that lead to SHP2 activation.

Mutations in PTPN11 have been identified in a variety of human diseases, such as Noonan syndrome, leopard spot syndrome, juvenile myelomonocytic leukemia, neuroblastoma, melanoma, acute myeloid leukemia and cancers of the breast, lungs and colon. SHP2 is an important downstream signaling molecule for a variety of receptor tyrosine kinases, including receptors of platelet-derived growth factor (PDGF-R), fibroblast growth factor (FGF-R) and epidermal growth factor (EGF-R). SHP2 is also an important downstream signaling molecule that activates the mitogen-activated protein (MAP) kinase pathway, which can lead to cell transformation (a necessary condition for cancer development). Knockdown of SHP2 significantly inhibits cell growth in lung cancer cell lines with SHP2 mutations or EML4/ALK translocations, as well as EGFR-amplified breast and esophageal cancers. SHP2 is also downstream of the activation of oncogenes for gastric cancer, anaplastic large cell lymphoma and glioblastoma.

PTPNll Mutations in Noonan syndrome (NS) and leopard spot syndrome (LS) cause LS (multiple pigmented plaque nevi syndrome, electrocardiogram conduction abnormalities, ocular hypertelorism, pulmonary stenosis, abnormal genitalia, growth retardation, sensorineural deafness) and NS (including heart defects, craniofacial deformities and congenital anomalies of short stature) and NS (including cardiac defects, craniofacial deformities and congenital anomalies of short stature). These two disorders are part of the family of autosomal dominant syndromes caused by germline mutations in components of the RAS/RAF/MEK/ERK mitogen-activated protein kinase pathway (required for normal cell growth and differentiation). Abnormal regulation of this pathway has profound effects, especially on cardiac development, leading to a variety of abnormalities, including valvuloseptal defects and/or hypertrophic cardiomyopathy (HCM). Perturbation of the MAPK signaling pathway has been established to be important for these disorders, and several candidate genes along this pathway have been identified in humans, including mutations in KRAS, NRAS, SOS1, RAF1, BRAF, MEK1, MEK2, SHOC2 and CBL. The most frequently mutated gene in NS and LS is PTPN11. Germline mutations in PTPN11 (SHP2) are found in about 50% of NS cases and in nearly all LS patients with certain features of NS. For NS, Y62D and Y63C in the protein are the most common mutations. These two mutations affect the catalytically inactive conformation of SHP2 without interfering with the binding of the phosphatase to its phosphorylated signaling partner.

Juvenile myelomonocytic leukemia (JMML) - somatic mutations in PTPN11 (SHP2) occur in approximately 35% of patients with JMML, a childhood myeloproliferative disorder (MPD). These gain-of-function mutations are usually point mutations in the N-SH2 domain or the phosphatase domain, which prevent self-inhibition between the catalytic and N-SH2 domains, resulting in SHP2 activity.

Acute myeloid leukemia - PTPN11 mutations have been already identified in ~10% of pediatric acute leukemias such as myelodysplastic syndrome (MDS), ~7% of B-cell acute lymphoblastic leukemia (B-ALL) and ~4% of acute myeloid leukemia (AML).

NS and leukemia mutations cause changes in amino acids located at the interface formed by the N-SH2 and PTP domains in the self-inhibitory SHP2 conformation, destroying inhibitory intramolecular interactions and resulting in hyperactivity of the catalytic domain.

SHP2 acts as a positive regulator in receptor tyrosine kinase (RTK) signaling. Cancers containing RTK alterations ($EGFR^{amp}$, $Her2^{amp}$, $FGFR^{amp}$, $Met^{amp}$, translocated/activated RTKs i.e. ALK, BCR/ABL) include esophageal cancer, breast cancer, lung cancer, colon cancer, gastric cancer, glioma, head and neck cancer.

Esophageal cancer (or esophagus cancer) is a malignant disease of the esophagus. Various subtypes exist, mainly squamous cell carcinoma (<50%) and adenocarcinoma. There is a high rate of RTK expression in esophageal adenocarcinoma and squamous cell carcinoma. Therefore, the SHP2 inhibitors of the present disclosure can be used in innovative therapeutic strategies.

Breast cancer is an important type of cancer and a leading cause of death in women where patients develop resistance to existing drugs. There are four main breast cancer subtypes, including luminal A, luminal B, Her21ik and triple negativeBasal-like. Triple-negative breast cancer (TNBC) is an aggressive breast cancer that lacks specific targeted therapies. Epidermal growth factor receptor I (EGFR) has emerged as a promising target in TNBC. Inhibition of Her2 and EGFR via SHP2 may be a promising treatment for breast cancer.

Lung cancer - NSCLC is currently a significant cause of cancer-related mortality, which accounts for about 85% of lung cancers (mainly adenocarcinomas and squamous cell carcinomas). While cytotoxic chemotherapy remains an important part of therapy, targeted therapies based on genetic alterations in tumors such as EGFR and ALK are more likely to benefit from targeted therapy.

Colon cancer - about 30% to 50% of colorectal tumors are known to have mutated (abnormal) KRAS, and BRAF mutations occur in 10 to 15% of colorectal cancers. For a subset of patients whose colorectal tumors have been shown to overexpress EGFR, these patients present a favorable clinical response to anti-EGFR therapy.

Gastric cancer is one of the most prevalent types of cancer. Abnormal expression of tyrosine kinases, as reflected by abnormal tyrosine phosphorylation in gastric cancer cells, is known in the art. Three receptor tyrosine kinases are frequently amplified in gastric cancer, namely c-met (HGF receptor), FGF receptor 2 and erbB2/neu. Therefore, destruction of different signaling pathways can promote the progression of different gastric cancers.

Neuroblastomas are pediatric tumors of the developing sympathetic nervous system that account for approximately 8% of childhood cancers. Genomic alterations in the anaplastic lymphoma kinase (ALK) gene have been proposed to contribute to neuroblastoma pathogenesis.

Squamous cell carcinoma of the head and neck (SCCHN) - high levels of EGFR expression are associated with poor prognosis and resistance to radiation therapy in a variety of cancers, most commonly squamous cell carcinoma of the head and neck (SCCHN). Blockade of EGFR signaling results in inhibitory receptor stimulation, decreased cell proliferation, invasion and metastasis. Therefore, in SCCHN, EGFR is an optimal target for new anticancer therapy.

The present disclosure relates to a compound capable of inhibiting the activity of SHP2. The present disclosure also provides methods for preparing the compound of the present disclosure and pharmaceutical formulations comprising the compound. Another aspect of the present disclosure relates to a method of treating a disease or disorder mediated by SHP2, which comprises the step of administering a therapeutically effective amount of an isotope-substituted compound represented by Formula I of the present disclosure to a patient in need thereof.

In some embodiments, the present disclosure relates to the method as described above, wherein the SHP2-mediated disease or disorder is a cancer selected from, but not limited to, the group consisting of JMML, AML, MDS, B-ALL, neuroblastoma, esophageal cancer, breast cancer, lung cancer, colon cancer, stomach cancer, head and neck cancer.

The compounds of the present disclosure may also be used to treat other diseases or disorders associated with abnormal SHP2 activity. Accordingly, as a preferred embodiment, the present disclosure relates to a method of treating a disease or disorder selected from the group consisting of NS, LS, JMML, AML, MDS, B-ALL, neuroblastoma, esophageal cancer, breast cancer, lung cancer, colon cancer, gastric cancer, head and neck cancer.

The SHP2 inhibitors of the present disclosure may be combined with another pharmacologically active compound or with two or more other pharmacologically active compounds, especially in the treatment of cancer. For example, an isotope-substituted compound of formula I of the present disclosure or a pharmaceutically acceptable salt thereof may be administered simultaneously, sequentially or separately in combination with one or more substances selected from the group consisting of chemotherapeutic agents, such as mitotic inhibitors, such as taxanes, vinca alkaloids, paclitaxel, docetaxel, vincristine, vinblastine, vinorelbine or vinflunine, other anticancer agents such as cisplatin, 5-fluorouracil or 5-fluoro-2-4(1H,3H)-pyrimidinedione (5FU), flutamide or gemcitabine.

The SHP2 inhibitor of the present disclosure can provide more excellent pharmacokinetic properties such as metabolic stability, half-life, in vivo clearance rate, and bioavailability through deuterium treatment at a specific site.

Certain combinations of compounds of the present disclosure may provide significant advantages in therapy, including synergistic activity.

In some embodiments, the present disclosure relates to the method as described above, wherein the compound is administered parenterally.

In some embodiments, the present disclosure relates to a method as described above, wherein the compound is administered intramuscularly, intravenously, subcutaneously, orally, via pulmonary delivery, intrathecally, topically or intranasally.

In some embodiments, the present disclosure relates to a method as described above, wherein the compound is administered systemically.

In some embodiments, the present disclosure relates to a method as described above, wherein the patient is a mammal.

In some embodiments, the present disclosure relates to a method as described above, wherein the patient is a primate.

In some embodiments, the present disclosure relates to a method as described above, wherein the patient is a human.

In some embodiments, the present disclosure relates to a method of treating a SHP2-mediated disease or disorder, which comprises the step of administering a combination of a therapeutically effective amount of a chemotherapeutic agent and a therapeutically effective amount of the compound represented by Formula I of the present disclosure to a patient in need thereof.

The present disclosure will be further described below in conjunction with specific examples. It should be understood that these examples are only used to illustrate the present disclosure and not to limit the scope of the present disclosure. In the following examples, the experimental methods without specific conditions are usually in accordance with conventional conditions, or in accordance with the conditions suggested by the manufacturer. Percentages and parts are by weight unless otherwise indicated.

The starting materials used in the following examples are purchased from chemical sellers such as Aldrich, TCI, Alfa Aesar, Bide, Energy and the like, or are synthesized by known methods.

The meaning represented by the English abbreviation in the following examples is described in the following table.

| | | | |
|---|---|---|---|
| $Ti(OEt)_4$ | tetraethyl titanate | DMF | N,N-dimethylformamide |
| $LiBH_4$ | lithium borohydride | $Na_2CO_3$ | sodium carbonate |
| TFA | trifluoroacetic acid | EtOH | ethanol |
| LDA | lithium N,N-diisopropylamine | $CBr_4$ | carbon tetrabromide |
| Pd (AmPhos)$_2Cl_2$ | bis(ditertbutyl(4-dimethylaminophenyl)phosphin e) dichloropalladium (II) | $Ph_3P$ | triphenylphosphine |
| $Cs_2CO_3$ | cesium carbonate | NBS | N-Bromosuccinimide |
| DMAc or DMA | N,N-dimethylacetamide | BPO | benzoyl peroxide |
| THF | tetrahydrofuran | TMP | 2,2,6,6-tetramethylpiperidine |
| DCM | dichloromethane | $PBr_3$ | phosphorus tribromide |
| $Ms_2O$ | methanesulfonic anhydride | $CCl_4$ | carbon tetrachloride |
| n-BuLi | n-butyl lithium | $N_2H_4$ | hydrazine |
| Dibal-H | diisobutyl aluminium hydride | $H_2SO_4$ | sulphuric acid |
| Dioxane | 1,4-dioxane | $POCl_3$ | phosphorusoxychloride |
| PPA | polyphosphoric acid | $Pd(OAc)_2$ | palladium acetate |
| $NaBH_4$ | sodium borohydride | EtONa | sodium ethanol |
| MeOH | methanol | $Pd_2(dba)_3$ | Tris(dibenzylideneacetone) dipalladium |
| $Et_3N$ or TEA | triethylamine | XantPhos | 4,5-Bis(diphenylphosphino)-9,9-di methylxanthene |
| DIPEA | N,N-diisopropylethylamine | $CH_3CN$ | acetonitrile |
| HCl | hydrogen chloride | $Boc_2O$ | di-tert-butyl dicarbonate |
| PMB-Br | 4-methoxybenzyl bromide | $K_2CO_3$ | potassium carbonate |

In the following examples, ice bath refers to -5 °C to 0 °C, room temperature refers to 10 ° C to 30 °C, and the reflux temperature generally refers to the reflux temperature of the solvent under atmospheric pressure. Reaction overnight generally refers to a time of 8-15 hours. The following examples without limiting the specific operating temperature are carried out at room temperature.

In the following examples, the separation and purification of the intermediate and final products are performed by normal-phase or reversed-phase chromatography column separation or other suitable methods. Normal-phase flash chromatography columns use ethyl acetate and n-hexane or methanol and dichloromethane as mobile phases. Reversed-phase preparative high-pressure liquid chromatography (HPLC) adopts C18 columns and is detected with UV 214 nm and 254 nm with mobile phase of A (water and 0.1% formic acid), B (acetonitrile) or mobile phase of A (water and 0.1% ammonium bicarbonate), B (acetonitrile).

In each example: LCMS Instrument: Pump Agilent 1260 UV Detector: Agilent 1260 DAD Mass Spectrometer API 3000

Chromatography column: Waters sunfire C18, 4.6×50mm, Sum
Mobile phase: A-H2O (0.1% HCOOH); B-acetonitrile NMR
Instrument: Bruker Ascend 400M (1HNMR: 400MHz; 13C NMR: 100 MHz).

In accordance with the method of WO2020094018, an intermediate A1: R-N-((S)-5,7-dihydrospiro[cyclopentadieno[b]pyridine-6,4'-piperidine]-5-yl)-2-methylpropane-2-sulfonamide was synthesized.

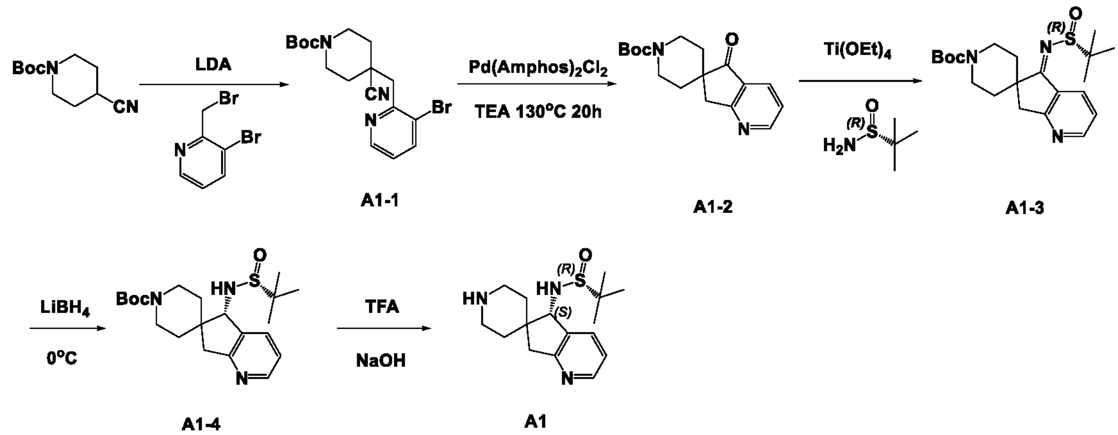


A1-1 A1-2 A1-3 A1-4 A1

Step 1: tert-butyl 4-cyanopiperidine-1-carboxylate (1.05g, 5 mmol) and THF (20 mL) were added to a dry 100 mL flask sequentially. Under nitrogen conditions, the mixture was cooled down to -78 °C, and then 2M LDA (3.3mL, 6.5mmol) was slowly added to the reaction mixture. The reaction mixture was allowed to react for 1 hour. Then 3-bromo-2-(bromomethyl)pyridine (1.24g, 5mmol) was added to the mixture, and the reaction mixture was allowed to further react for 2 hours. After the completion of the reaction, a saturated ammonium chloride solution (15mL) was added to quench the reaction, and the reaction product was extracted with ethyl acetate (3×30mL). The organic layers were mixed and washed with saturated brine, dried with anhydrous sodium sulfate, filtered and concentrated under reduced pressure. The residue was purified by silica gel chromatography (ethyl acetate/petroleum ether with 0 to 30% gradient), to obtain a white solid tert-butyl 4-((3-bromopyridin-2-yl)methyl)-4-cyanopiperidine-1'-carboxylate (A1-1,1.40g, yield: 75%).

Step 2: Under nitrogen protection, tert-butyl 4-((3-bromopyridin-2-yl)methyl)-4-cyanopiperidine-1'-carboxylate (A1-1, 379mg, 1mmol), triethylamine (404mg, 4mmol), bis-di-tert-butyl-(4-dimethylaminophenyl)phosphine dichloro-palladium(II) Pd(AmPhos)$_2$Cl$_2$ (71mg, 0.1mmol) and DMA:H2O=10:1 ( 6 mL) were added sequentially to a dry 25 mL single-mouth flask, then stirred for reaction at 130 °C for 18 h. After the completion of the reaction, the obtained residue was filtered and concentrated under reduced pressure. The resultant residue was purified by silica gel chromatography (ethyl acetate: petroleum ether with 0 to 50% gradient) to give a yellow solid tert-butyl 5-oxo-5,7-dihydrospiro[cyclopentadieno [b] pyridine-6,4'-piperidine]-1'-carboxylate (A1-2,180mg, yield: 60%) LCMS: m/z 303.1 [M+H]+.

Step 3: tert-butyl 5-oxo-5,7-dihydrospiro[cyclopentadieno[b]pyridine-6,4'-piperidine]-1'-carboxylate (A1-2, 0.302g, 1 mmol), tetraethyl titanate (1.37g, 6mmol), (R)-(+)-tert-butylsulfinamide (0.480g, 4mmol) were added sequentially to a dry 100 mL single-mouth flask, stirred for reaction under heat reflux for 15 hours. After the reaction mixture was cooled to room temperature, saturated brine (15 mL) was added to the reaction residue, after which the resulting mixture was stirred for 15 minutes and then filtered through diatomaceous earth. The aqueous mixture was extracted with ethyl acetate (3x300mL). The organic phases were dried with $Na_2SO_4$, filtered, and the volatiles were removed under reduced pressure. The resulting residue was purified by silica gel chromatography (ethyl acetate: petroleum ether with 0 to 50% gradient) to obtain a yellow solid tert-butyl (R,Z)-5-((tert-butylsulfinyl)imino)-5,7-dihydrospiro[cyclopentadieno[b]pyridine-6,4'-piperidine]-1'-carb oxylate (**A1-3**, 0.333g, yield: 82%). LCMS: m/z 406.1 [M+H]+.

Step 4: tert-butyl (R,Z)-5- ((tert-butylsulfinyl)imino)-5,7-dihydrospiro[cyclopentadieno[b] pyridine-6,4'-piperidine]-1'-carboxylate **(A1-3,** 0.20g, 0.491 mmol) and THF (50 mL) were added sequentially to a dry 100 mL single-mouth flask. After the reaction mixture was cooled to 0 °C, lithium borohydride (0.018g, 0.737 mmol) was added. The resulting mixture was continued to be stirred for reaction for 1 h. Methanol was added slowly to quench excess borohydride and the reaction product was filtered, concentrated, and volatiles were removed under reduced pressure. The resulting residue was purified by silica gel chromatography (ethyl acetate: petroleum ether with 0 to 80% gradient) to obtain a white solid tert-butyl (S)-5-((R)-tert-butylsulfinamide)-5,7-dihydrospiro[cyclopentadieno[b]pyridine-6,4'-piperidine]-1'-carboxylate (**A1-4**, 0.130g, yield: 65%). LCMS: m/z 408.1 [M+H]+.

Step 5: tert-butyl (S)-5-(R)-tert-butylsulfinamide)-5,7-dihydrospiro [cyclopentadieno[b] pyridine-6,4'-piperidine]-1'-carboxylate (A1-4, 0.100g, 0.245mmol), dichloromethane (5mL), trifluoroacetic acid (1mL) were added sequentially to a dry 50 mL single-mouth flask. The resulting mixture was stirred at room temperature for 1 h. Saturated aqueous solution of $Na_2CO_3$ was added until pH was 7 and the aqueous mixture was extracted with DCM (3x30mL). The combined organic phases were washed with saturated brine, dried with $Na_2SO_4$ and filtered. Volatiles were removed under reduced pressure. After the reaction mixture was cooled, a colorless oil R-N-((S)-5,7-dihydrospiro [cyclopentadieno[b]pyridine-6,4'-piperidine]-5-yl)-2-methylpropane-2-sulfinamide (**A1**, 0.056g, yield: 75%) was obtained. LCMS: m/z 308.1 [M+H]+.

Synthesis of Intermediate A2: (R)-N-((S)-5,7-dihydrospiro [cyclopentadieno[b]pyridine-6,4'-piperidine]-5-yl-5-deuterium)-2-methylpropane-2-sulfinamide

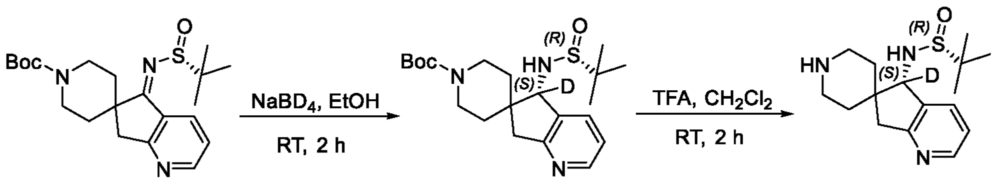


A1-3 A2-1 A2

Step 1: Sodium Borodeuteride (186 mg, 4.44 mmol) was added to an ethanol solution (20 mL) of (R,Z)-5-((tert-butylsulfinyl)imino-tert-butyl)-5,7-dihydrospiro[cyclopenta[b]pyridine-6,4'-piperi dine]-1'-carboxylate (600 mg, 1.48 mmol) and reacted at room temperature for 2 hours. TLC and LCMS showed the completion of the reaction. Acetic acid (1 mL) was added to quench the reaction. The reaction liquid was diluted with ethyl acetate (100 mL) and stirred at room temperature for 1 hour after the addition of saturated aqueous solution of sodium chloride. After separation, the aqueous phase was extracted with ethyl acetate (100 mL). The organic phases were combined, washed with saturated brine, dried, filtered, and concentrated to dry under reduced pressure. After purification by column chromatography (petroleum ether: ethyl acetate=1:1), a yellow oily liquid tert-butyl (S)-5-((R)-tert-butylsulfinyl)amino)-5,7-dihydrospiro[cyclopenta[b]pyridine-6,4'-piperidine]- 1'-carboxylate-5-deuterium (400 mg, yield 66.2%) was obtained.

LCMS: m/z: 409.5 [M+H]+.

Step 2: Trifluoroacetic acid (3 mL) was added to a solution of (5-(((R)-tert-butylsulfinyl)amino)tert-butyl-5,7-dihydrospiro[cyclopenta[b]pyridine-6,4'-piperid ine]-1'-carboxylate-5-d (816 mg, 2.0 mmol) in dichloromethane (10 mL) and reacted at room temperature for 2 hours. TLC and LCMS showed the completion of reaction. The reaction liquid was concentrated to dry under reduced pressure to obtain a white solid (R)-N-((S)-5,7-dihydrospiro[cyclopentadieno[b]pyridine-6,4'-piperidine]-5-yl-5-deuterium)-2-methylpropane-2-sulfinamide. LCMS: m/z: 309.5 [M+H]+.

Synthesis of Intermediates A3-A5:

In accordance with the synthesis method of Intermediate A1, different deuterated starting materials were used to obtain Intermediates A3, A4 and A5.

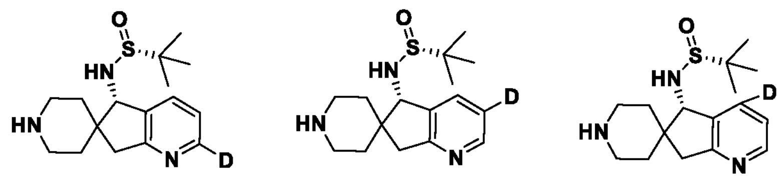

A3 A4 A5

A3, LCMS: m/z 309.1 $[M+H]^+$
A4, LCMS: m/z 309.1 $[M+H]^+$
A5, LCMS: m/z 309.1 $[M+H]^+$

Synthesis of Intermediate A6:

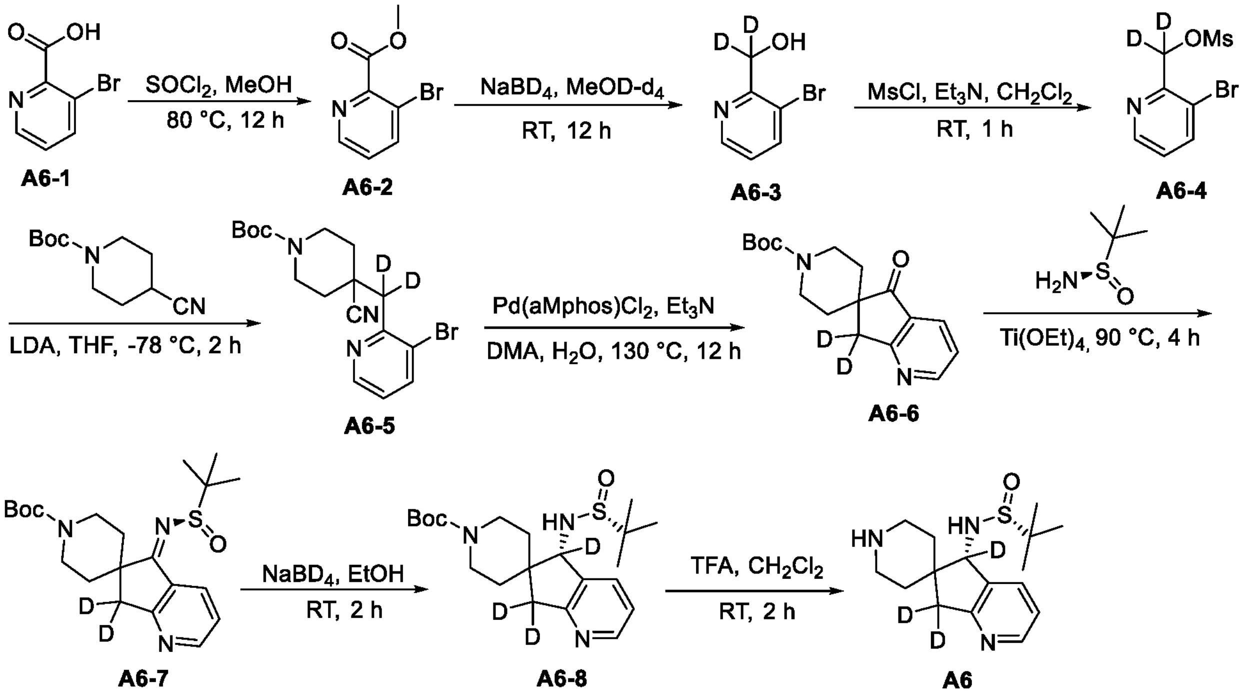


Step 1: Sulfoxide chloride (8.85 g, 74.4 mmol) was added to a solution of 3-bromopicolinic acid (A6-1, 5.0 g, 24.8 mmol) in methanol (80 mL) under ice bath and reacted at 80 °C for 12 h. The reaction product was concentrated under reduced pressure to obtain a crude product, which was diluted with ethyl acetate (180 mL), neutralized with saturated aqueous solution of sodium bicarbonate (150 mL) to pH = 9, and separated. After the reaction mixture was dried with sodium anhydrous sulfate and filtered, the filtrate was concentrated to dry under reduced pressure and purified by silica gel chromatography (ethyl acetate: petroleum ether with 0 to 30% gradient) to produce a pale yellow oily liquid methyl 3-bromopyridinecarboxylate **(A6-2,** 4.8 g, yield 89.8%). LCMS: m/z: 217 [M+H]+.

Step 2: Sodium Borodeuteride (186 mg, 4.44 mmol) was added to a solution of methyl 3-bromopyridinecarboxylate **(A6-2,** 300 mg, 1.39 mmol) in deuterated methanol (8 mmol) at 0 °C and reacted at room temperature for 12 hours. TLC and LCMS showed the completion of reaction. The reaction liquid was diluted with ethyl acetate (100 mL) and stirred at room temperature for 1 hour after the addition of saturated aqueous solution of sodium chloride. After separation, the aqueous phase was extracted with ethyl acetate (100 mL). The organic phases were combined, washed with saturated brine, dried, filtered, and concentrated to dry under reduced pressure. After purification by column chromatography, a white solid (3-bromopyridine-2-yl) methane-d2-ol **(A6-3,** 130 mg, yield 49.2%) was obtained.

LCMS: m/z: 191 [M+H]+.

Step 3: Triethylamine (959 mg, 9.48 mmol) and methylsulfonyl chloride (723 mg, 6.32 mmol) were added to a solution of (3-bromopyridine-2-yl) methane-d2-ol **(A6-3,** 600 mg, 3.16 mmol) in dichloromethane (30 mL) under argon protection and stirred for reaction at room temperature for 1 hour. The reaction liquid was poured into ice water (20 mL), extracted with dichloromethane (2×150 mL). The organic phases were combined, washed with saturated brine (100 mL), dried with anhydrous sodium sulfate, and filtered. The filtrate was concentrated to dry under reduced pressure to obtain a yellow oily liquid (3-bromopyridine-2-yl) methyl-d2-methylsulfonate **(A6-4,** 210 mg, yield 24.8%).

LCMS: m/z: 269 $[M+H]^+$.

Step 4: A solution of lithium diisopropylamide in tetrahydrofuran (2 M, 1.5 mL, 3.0 mmol) was added dropwise to a solution of tert-butyl 4-cyanopiperidine-1-carboxylate (420 mg, 2 mmol) in tetrahydrofuran (10 mL) under argon protection at -78 °C and stirred for reaction at this temperature for 0.5 hours. A solution of (3-bromopyridine-2-yl)methyl-d2-methylsulfonate (A6-4, 268 mg, 1 mmol) in THF (15 mL) was added dropwise to the reaction liquid and reacted for 2 hours at -78 °C. The reaction liquid was poured into an aqueous solution of saturated ammonium chloride (20 mL), extracted with dichloromethane (2×50 mL). The organic phases were combined, washed with saturated brine (100 mL), dried with anhydrous sodium sulfate, and filtered. The filtrate was concentrated to dry under reduced pressure to obtain a yellow oily liquid tert-butyl 4-((3-bromopyridine-2-yl)methyl-d2)-4-cyanopiperidine-1-carboxylate (A6-5, 110 mg, yield 28.8%).

LCMS: m/z: 382/384$[M+H]^+$.

Step 5: Pd(aMphos)$Cl_2$ (3.7 g, 5.25 mmol), triethylamine (15.9 g, 157.5 mmol) were added to a solution of tert-butyl 4-((3-bromopyridine-2-yl)methyl-d2)-4-cyanopiperidine-1-carboxylate **(A6-5,** 20.0 g, 52.5 mmol) in water (50 mL)

and N,N-dimethylacetamide (150mL) and reacted for 12 hours at 130°C after nitrogen displacement. The reaction liquid was diluted with ethyl acetate (1000 mL) and stirred at room temperature for 1 hour after the addition of saturated aqueous solution of sodium chloride. After separation, the aqueous phase was extracted with ethyl acetate (600 mL). The organic phases were combined, washed with saturated brine, dried, filtered, and concentrated to dry under reduced pressure. After purification by column chromatography, a yellow solid tert-butyl 5-oxo-5,7-dihydrospiro[cyclopenta[b]pyridine-6,4'-piperidine]-1'- carboxylate-7,7-d2 **(A6-6,** 3.5 g, yield 22.0 %) was obtained.

LCMS: m/z: 305[M+H]$^+$.

Step 6: (R)-2-Methylpropane-2-sulfinamide (200 mg, 1.66 mmol) was added to a solution of tert-butyl 5-oxo-5,7-dihydrospiro[cyclopenta[b]pyridine-6,4'-piperidine]-1'-carboxylate-7,7-d2 **(A6-6,** 420 mg, 1.38 mmol) in tetraethyl titanate (20 mL) and reacted for 4 hours at 90 °C after nitrogen displacement. The reaction liquid was poured into water (100mL) and extracted with ethyl acetate (800mL). The organic phases were combined, washed with saturated brine, dried, filtered, and concentrated to dry under reduced pressure. After purification by column chromatography, a yellow solid tert-butyl (R,Z)-5-((tert-butylsulfinyl)imino)-5,7-dihydrospiro [cyclopenta[b]pyridine-6,4'-piperidine]-1'-carboxylate-7,7-d2 **(A6-7,** 355 mg, yield 63.1 %).

LCMS: m/z: 408[M+H]$^+$.

Step 7: Sodium borodeuteride (40 mg, 0.96 mmol) was added to a solution of tert-butyl (R,Z)-5-((tert-butylsulfinyl)imino)-5,7-dihydrospiro[cyclopenta[b]pyridine-6,4'-piperidine]-1'-c arboxylate-7,7-d2 **(A6-7,** 260 mg, 0.64 mmol) in ethanol (10 mL) and reacted for 2 hours at room temperature. TLC and LCMS showed the completion of the reaction. Acetic acid (0.5 mL) was added to quench the reaction. The reaction liquid was diluted with ethyl acetate (100 mL) and stirred at room temperature for 1 hour after the addition of saturated aqueous solution of sodium chloride. After separation, the aqueous phase was extracted with ethyl acetate (100 mL). The organic phases were combined, washed with saturated brine, dried, filtered, and concentrated to dry under reduced pressure. After purification by column chromatography, a yellow oily liquid tert-butyl (S)-5-((R)-tert-butylsulfinyl)amino)-5,7-dihydrospiro [cyclopenta[b]pyridine-6,4'-piperidine]-1'-carboxylate-5,7,7-d3 **(A6-8,** 155 mg, yield 59.2%) was obtained.

LCMS: m/z: 411[M+H]$^+$.

Step 8: Trifluoroacetic acid (3 mL) was added to a solution of tert-butyl (S)-5-((R)-tert-butylsulfinyl)amino)-5,7-dihydrospiro[cyclopenta[b]pyridine-6,4'-piperidine]-1'-carboxylate-5,7,7-d3 **(A6-8,** 410 mg, 1 mmol) in dichloromethane (10 mL) and reacted at room temperature for 2 hours. TLC and LCMS showed the completion of reaction. The reaction liquid was concentrated to dry under reduced pressure to obtain a white solid (R)-N-((S)-5,7-dihydrospiro[cyclopenta[b]pyridine-6,4'-piperidine]-5-yl-5,7,7-d3)-2-methylpro pane-2-sulfinamide **(A6,** 350 mg, yield 100%). LCMS: m/z: 311 [M+H]$^+$.

Synthesis of Intermediate B1: 5-chloro-8-iodoimidazo[1,2-c]pyrimidine

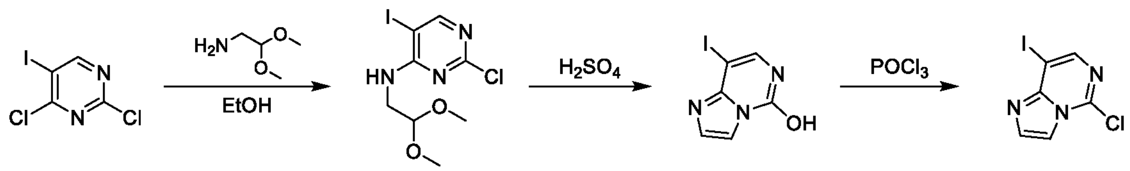


B1-1 B1-2 B1

Step 1: 2,4-Dichloro-5-iodopyrimidine (1.37g, 5mmol) and 2,2-dimethoxyethylamine (8.4g, 10mmol) and absolute ethanol (50mL) were successively added to a dry 100 mL flask. Then, triethylamine (1.01g, 10mmol) was slowly added to the reaction mixture under a condition of 0 °C nitrogen and then the mixture was stirred for reaction at room temperature for 10 hours. After the completion of the reaction, the reaction liquid was concentrated under vaccum and 15mL of water was added to the resulting concentrate. The mixture was extracted with dichloromethane (3×50mL) and washed with saturated brine. The organic layers were mixed and dried with anhydrous sodium sulfate, filtered and concentrated to obtain a white solid 2-chloro-N-(2,2-dimethoxyethyl)-5-iodopyrimidine-4-amine (**B1-1**, 1.46g, yield: 85%).

LC-MS: m/z 344.2 [M+H]$^+$.

Step 2: 2-chloro-N-(2,2-dimethoxyethyl)-5-iodopyrimidine-4-amine **(B3-1,** 1.03g, 3mmol) and 10 mL concentrated sulfuric acid were successively added to a dry 100 mL flask. Under nitrogen conditions, the mixture was heated to 65 °C and stirred for reaction for 2 hours. After the completion of the reaction, the reaction liquid was cooled to room temperature. The mixture was slowly poured into ice water, and then the pH was adjusted to about 6-7 with 4 M of NaOH solution. After filtration, an off-white solid 8-iodoimidazo[1,2-c]pyrimidine-5-ol (B1-2,407 mg, yield 52%) was obtained.

LC-MS: m/z 262.2 [M+H]$^+$.

Step 3: 8-iodoimidazo[1,2-c]pyrimidine-5-ol **(B3-2,** 522mg, 2mmol) and phosphorus oxychloride (8mL) were successively added to a dry 50 mL single-mouth flask. Under the protection of nitrogen, N, N-diisopropylethylamine (1 mL) was slowly added dropwise, after which the mixture was heated to 120 °C and stirred for 5 hours. After the completion of the reaction, the reaction liquid was cooled to room temperature and concentrated under vacuum. Then a saturated sodium bicarbonate solution was added to quench the reaction. The reaction liquid was extracted with ethyl acetate (3×40mL), dried with anhydrous sodium sulfate, filtered and concentrated. The resulting residue was purified by silica gel chromatography (ethyl acetate: petroleum ether with 0 to 30% gradient) to give a pale yellow solid 5-chloro-8-iodimidazo[1,2-c] pyrimidine (**B1**, 360mg, yield: 55%).

LC-MS: m/z 280.1 $[M+H]^+$.

Synthesis of Intermediate B1: 5-chloro-8-iodimidazo[1,2-c] pyrimidine-7-deuterium

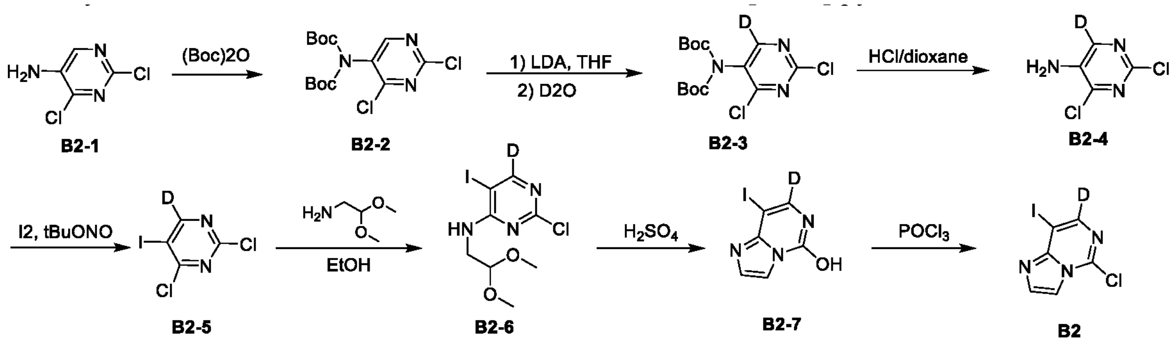

B2-1 B2-2 B2-3 B2-4 B2-5 B2-6 B2-7 B2

In accordance with the synthesis method of Intermediate B1, the Intermediate B2 5-chloro-8-iodoimidazo[1,2-c]pyrimidine-7-deuterium, LC-MS:m/z 281.1 [M+H]+ was synthesized from B2-1. B2 can also be synthesized from other starting intermediates.

Synthesis of Intermediate **B-B4**:

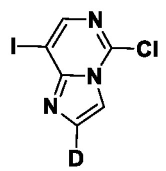

B3

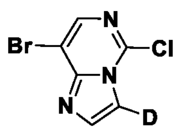

B4

In accordance with the synthesis method of Intermediate B1, the Intermediates B3, B4 were synthesized from suitable material.

B3, 5-chloro-8-iodoimidazo[1,2-c]pyrimidine-2-deuterium, LCMS: m/z 281.1 $[M+H]^+$

B4, 5-chloro-8-bromoimidazo[1,2-c]pyrimidine-3-deuterium, LCMS: m/z 232.9 $[M+H]^+$

In accordance with the method of WO2020094018, Intermediate **C1**: sodium 2-amino-3-chloropyridine-4-thiolate was synthesized.

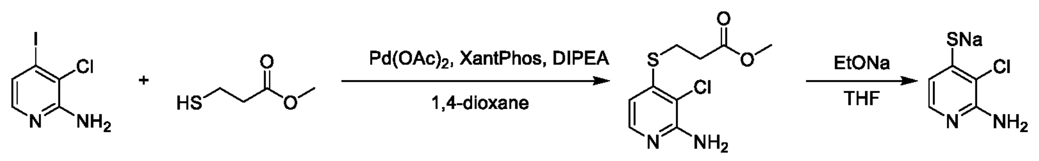


C1-1 C1

Step 1: 3-chloro-4-iodopyridine-2-amine (2.5g, 9.82mmol, 1.0eq), XantPhos (341mg, 0.59mmol. 0.06eq), palladium acetate (110mg, 0.49mmol, 0.05eq), DIPEA (3.25mL, 19.6mmol, 2.0 q), methyl 3-mercaptopropionate (1.19mL, 10.8 mmol, 1.1eq) and 1,4-dioxane (32.5 mL) was successively added to a dry 100mL round bottom three-necked flask, replaced with nitrogen three times under agitation, then heated to 100°C and reacted for three hours. After the completion of the reaction, the reaction liquid was cooled to room temperature, diluted with ethyl acetate (50mL) and filtered under reduced pressure. The filter cake was washed with ethyl acetate (25mL). The resulting filtrate was concentrated under vacuum and the resulting residue was purified by silica gel chromatography (ethyl acetate: petroleum ether with 0 to 30% gradient) to give a yellow solid methyl 3-((2-amino-3-chloropyridin-4-yl)thio)propionate (C1-1, 2.0g, yield: 78%).

Step 2: The compound C1-1 (2g, 8.11mmol, 1.0eq) was dissolved in tetrahydrofuran (28mL) in a dry 100 mL round-bottom three-necked flask. Under nitrogen protection, sodium ethanol (2.9g, 8.51mmol, 1.05 eq, 20% wt) was added dropwise to the reaction liquid at room temperature, and then stirred for one hour. After the completion of the reaction, the reaction liquid was diluted with dichloromethane (60mL), sonicated for 5 minutes, and filtered under reduced pressure. The filter cake was vacuum-dried to obtain a yellow solid sodium 2-amino-3-chloropyridine-4-thiolate (C1, 1.4g, yield: 89%).

Synthesis of Intermediates **C2-C5:** in accordance with the synthesis method of Intermediate C1, Intermediates **C2-C5** were synthesized from suitable materials:

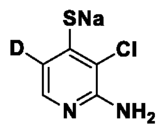

**C2**

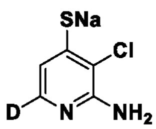

**C3**

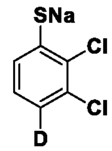

**C4**

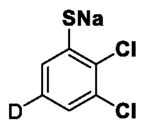

**C5**

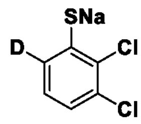

**C6**

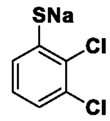

**C7**

Intermediate **C2,** sodium 2-amino-3-chloropyridine-4-thiolate-5-deuterium;
Intermediate **C3,** sodium 2-amino-3-chloropyridine-4-thiolate-6-deuterium;
Intermediate **C4,** sodium 2, 3-dichlorophenyl thiolate-4-deuterium;
Intermediate **C5,** sodium 2, 3-dichlorophenyl thiolate-5-deuterium;
Intermediate **C6,** sodium 2, 3-dichlorophenyl thiolate-6-deuterium;
Intermediate **C7,** sodium 2, 3-dichlorophenyl thiolate.

**Example 1: Synthesis of Compound 1**

**(S)-1'-(8-((2-amino-3-chloropyridin-4-yl)thio)imidazo[1,2-c]pyrimidin-5-yl)-5,7-dihyd rospiro[cyclopentadieno[b]pyridine-6,4'-piperidine]-5-amine**

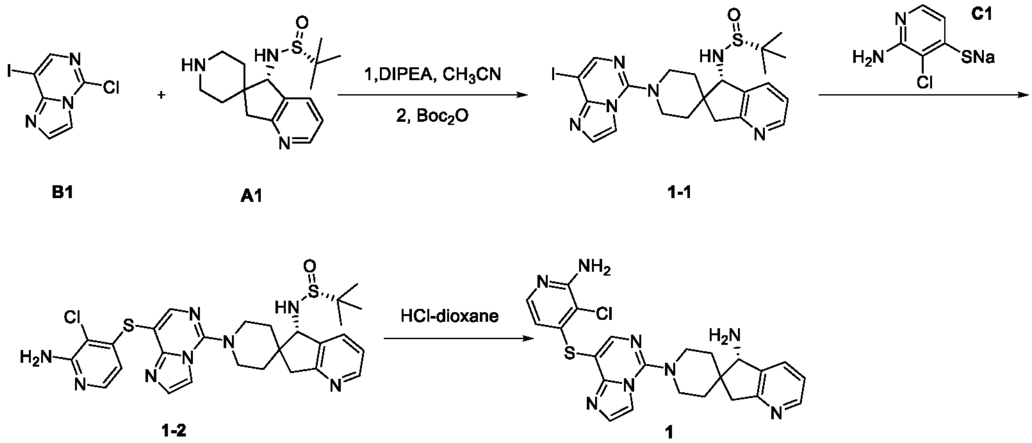


B1 A1 1-1

1-2 1

Step 1: B1 (1.37g, 4.9mmol), A1 (1.35g, 4.9mmol) and DIPEA (4.86mL, 29.41mmol) were added sequentially to a 25mL single-mouth flask containing 3mL acetonitrile, and then stirred for reaction at 80 °C for 2 hours. After the completion of the reaction, the reaction liquid was cooled to room temperature, and then added with BoczO (1.6g,

7.35mmol, 1.5eq), heated to 50 °C until the reaction was complete. The residue obtained by concentrating the reaction liquid under reduced pressure was purified by silica gel chromatography (ethyl acetate/petroleum ether with 0 to 100% gradient) to give a yellow solid 1-1 (1.7g, yield: 63.4%). LC-MS: m/z = 547.0 [M +H+]

Step 2: 1-1 (1.7 g, 3.11 mmol), sodium 2-amino-3-chloropyridine-4-thiolate (C1, 596 mg, 3.27 mmol), Pd2(dba)3 (285 mg, 0.311 mmol), Xantphos (360 mg, 0.622 mmol), DIPEA (804 mg, 6.22 mmol) and 1,4-dioxane solution (30 mL) were added sequentially to a microwave reaction flask under nitrogen protection. The mixture was microwaved to 100 °C under nitrogen protection and stirred for reaction for 3 hours. After the completion of the reaction, the reaction liquid was cooled to room temperature and filtered. The residue obtained by concentrating the reaction liquid under reduced pressure was purified by silica gel chromatography (ethyl acetate/methanol with 0 to 10% gradient) to obtain (S)-tert-butyl(1'-(8-((2-amino-3-chloropyridin-4-yl)thio)imidazo[1,2-c]pyrimidine-5-yl)-5,7-dihydrospiro[cyclopentadieno[b]py ridine-6,4'-piperidine]-5-yl)-carbonate (1-2,1.2g, 66.7%).

Step 3: TFA (5mL) was added to a solution of (S)-tert-butyl(1'-(8-((2-amino-3-chloropyridin-4-yl)thio)imidazo[1,2-c]pyrimidine-5-yl)-5,7-dihydrospiro[cyclopentadieno[b]py ridine-6,4'-piperidine]-5-yl)-carbonate **(1-2,** 1.2g, 2.07mmol) in dichloromethane (SmL) at 0 °C under nitrogen protection and stirred for reaction at room temperature for 1 hour. TLC and LCMS showed the completion of the reaction. The reaction liquid was concentrated under reduced pressure. The residue was then dissolved in a mixed solution of dichloromethane/methanol, and the pH was adjusted to neutral with $NaHCO_3$. After purification by silica gel column, (S)-1'-(8-((2-amino-3-chloropyridin-4-yl)thio)imidazo[1,2-c] pyrimidine-5-yl)-5,7-dihydrospiro [cyclopentadieno[b]pyridine-6,4'-piperidine]-5-amine (Compound 1,400 mg, yield: 40.0%) was obtained.

$^1H$ NMR (400 MHz, DMSO-*d6*) δ 8.35 (d, *J* = 4.0 Hz, 1H), 8.03 (s, 1H), 7.83 (d, *J* = 1.2 Hz, 1H), 7.72 (d, *J* = 7.6 Hz, 1H), 7.56 (dd, *J* = 10.4, 3.4 Hz, 2H), 7.20 (dd, *J* = 7.6, 5.2 Hz, 1H), 6.33 (s, 2H), 5.80 (d, *J* = 5.4 Hz, 1H), 4.02 (s, 1H), 3.95 (dd, *J* = 11.6, 7.6 Hz, 2H), 3.31 (d, *J* = 13.6 Hz, 2H), 3.15 (d, *J* = 16.4 Hz, 1H), 2.83 (d, *J* = 16.4 Hz, 1H), 2.00 (tt, *J* = 12.4, 6.4 Hz, 2H), 1.64 (d, *J* = 13.2 Hz, 1H), 1.48 (dd, *J* = 13.6, 6.4 Hz, 1H), 1.34-1.29 (m, 2H);
LCMS: m/z 479.0 $[M+H]^+$.

### Example 2: Synthesis of Compound 10

(S)-1'-(8-((2-amino-3-chloropyridin-4-yl)thio)imidazo[1,2-c]pyrimidine-5-yl)-5,7-dihydros piro[cyclopentadieno[b]pyridine-6,4'-piperidine]-5-deuterium-5-amine

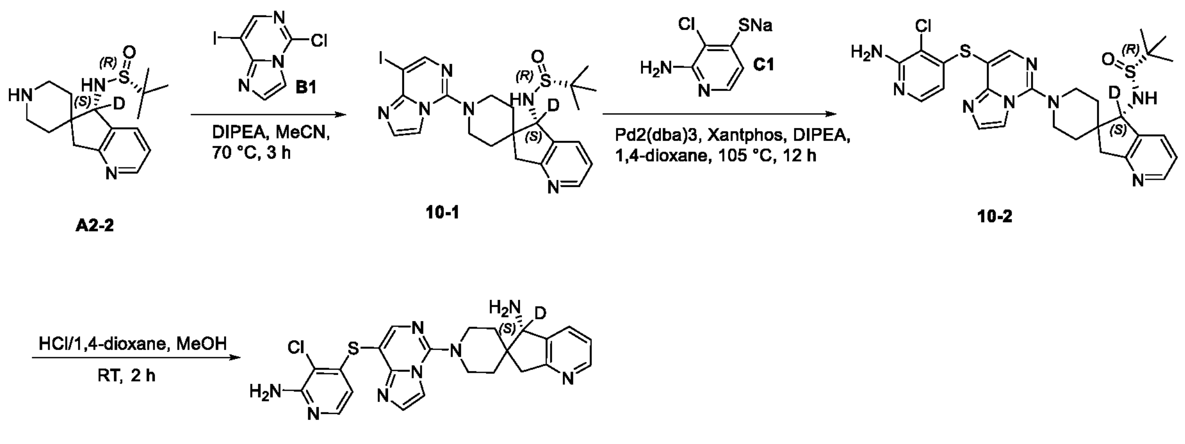


A2-2 10-1 10-2

10

Step 1: N,N-Diisopropylethylamine (500 mg, 3.87 mmol) was added to a solution of (R)-N-((S)-5,7-dihydrospiro[cyclopentadieno[b]pyridin-6,4'-piperidine]-5-yl-5-deuterium)-2-m ethylpropane-2-sulfinamide (A2-2, 400 mg, 1.29 mmol), 5-chloro-8-iodoimidazo[1,2-c]pyrimidine (B1, 359 mg, 1.29 mmol) in acetonitrile (10 mL) and reacted at 70 °C for 3 h. The reaction liquid was diluted with ethyl acetate (100 mL) and stirred at room temperature for 1 hour after the addition of saturated aqueous solution of sodium chloride. After separation, the aqueous phase was extracted with ethyl acetate (100 mL). The organic phases were combined, washed with saturated brine, dried, filtered, and concentrated to dry under reduced pressure. After purification by column chromatography (dichloromethane: methanol=20:1), a yellow solid of (R)-N-((S)-1'-(8-iodoimidazo[1,2-c]pyrimidin-5-yl)-5,7-dihydrospiro[cyclopenta[b]pyridine-6,4'-piperidine]-5-yl-5-d)-2-methylpropane-2-sulfinami de **(10-1,** 610 mg, yield 85.8%) was obtained. LCMS: m/z: 552.1 $[M+H]^+$.

Step 2: Tris(dibenzylideneacetone)dipalladium (50 mg, 0.05 mmol), N,N-diisopropylethylamine (140 mg, 1.09

mmol), Xantphos (63 mg, 0.11 mmol) were added to a solution of (R)-N-((S)-1'-(8-iodoimidazo[1,2-c]pyrimidin-5-yl)-5,7-dihydrospiro[cyclopenta[b] pyridine-6,4'-piperidine]-5-yl-5-d)-2-methylpropane-2-sulfonamide **(10-1,** 300 mg, 0.54 mmol), sodium 2-amino-3-chloropyridine-4-thiosulfate (110 mg, 0.60 mmol) in dioxane (10 mL) and reacted for 12 hours at 105 °C after nitrogen replacement. The reaction liquid was diluted with ethyl acetate (100 mL) and stirred at room temperature for 10 mins after the addition of saturated aqueous solution of sodium chloride. After separation, the aqueous phase was extracted with ethyl acetate (100 mL). The organic phases were combined, washed with saturated brine, dried, filtered, and concentrated to dry under reduced pressure. After purification by column chromatography (dichloromethane: methanol=20:1), a yellow solid of (R)-N-((S)-1'-(8-(2-amino-3-chloropyridin-4-yl)thio)imidazo[1,2-c]pyrimidin-5-yl)-5,7-dihydrospiro[c yclopenta[b]pyridine-6,4'-piperidine]-5-yl-5-deuterium)-2-methylpropane-2-sulfinamide **(10-2,** 210 mg, yield 66.1%) was obtained.

LCMS: m/z: 584.2 $[M+H]^+$.

Step 3: Hydrochloric acid/1,4-dioxane (4 M, 5 mL) was added to a solution of (R)-N-((S)-1'-(8-(2-amino-3-chloropyridin-4-yl)thio)imidazo[1,2-c]pyrimidin-5-yl)-5,7-dihydrospiro[c yclopenta[b]pyridine-6,4'-piperidine]-5-yl-5-deuterium)-2-methylpropane-2- sulfinamide **((10-2,** 150 mg, 0.26 mmol) in methanol (8 mL) and reacted for 2 hours at room temperature after nitrogen replacement. TLC and LCMS showed the completion of the reaction. The reaction liquid was concentrated to dry under reduced pressure. The crude product was dissolved with ethyl acetate (30 mL) and stirred at room temperature for 10 mins after the pH was adjusted to 9 by saturated aqueous solution of sodium bicarbonate. After separation, the aqueous phase was extracted with ethyl acetate (100 mL). The organic phases were combined, washed with saturated brine, dried, filtered, and concentrated to dry under reduced pressure. After purification by column chromatography, a white solid of (S)-1'-(8-(((2-amino-3-chloropyridin-4-yl)thio)imidazo[1,2-c]pyrimidine-5-yl)-5,7-dihydrospiro [cyclopenta[b]pyridine-6,4'-piperidine]-5-d-5-amine (100 mg, yield 81.0%).

LCMS: m/z: 480.1 $[M+H]^+$.

$^1H$ NMR (400 MHz, DMSO-$d_6$): 8.36 (d, $J$ = 4.8 Hz, 1H), 8.03 (s, 1H), 7.83 (d, $J$ = 1.2 Hz, 1H), 7.74 (d, $J$ = 8.0 Hz, 1H), 7.58 (s, 1H), 7.54 (d, $J$ =5.6 Hz, 1H), 7.23-7.20 (dd, $J$ = 7.6, 5.2 Hz, 1H, 1H), 6.34 (s, 2H), 5.80 (d, $J$ =5.6 Hz, 1H), 3.97-3.94 (dd, $J$ = 11.6, 7.6 Hz, 2H), 3.39-3.37 (d, $J$ = 13.6 Hz, 2H), 3.15 (d, $J$ =16.4 Hz, 1H ), 2.86 (d, $J$ =16.4 Hz, 1H ), 2.03-1.94 (m, 2H), 1.64 (d, $J$ =13.6 Hz, 1H ), 1.36 (d, $J$ =13.6 Hz, 1H ).

**Example 3: Synthesis of Compound 12**

(S)-1'-(8-((2-amino-3-chloropyridin-4-yl)thio)imidazo[1,2-c]pyrimidin-5-yl)-5,7-dihydros piro[cyclopentadieno[b]pyridine-6,4'-piperidine]-5,7,7-d3-5-amine

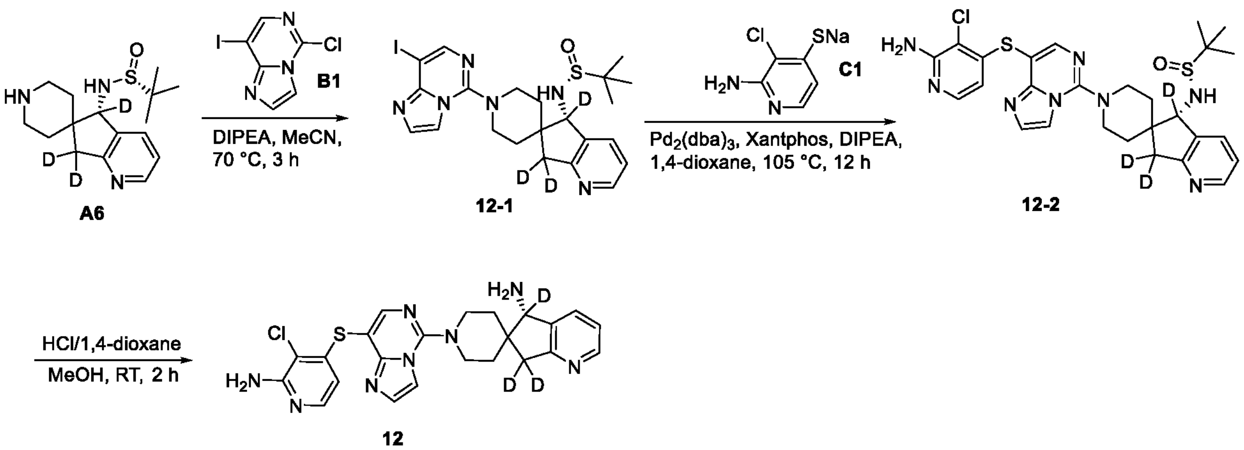

A6 12-1 12-2 12

In accordance with the synthesis method of Compound 10, Compound 12 was synthesized from A6.

LCMS: m/z: 482.2$[M+H]^+$.

$^1H$ NMR (400 MHz, DMSO-$d_6$): 8.36 (d, $J$ = 4.8 Hz, 1H), 8.03 (s, 1H), 7.83 (d, $J$ = 1.2 Hz, 1H), 7.74 (d, $J$ = 8.0 Hz, 1H), 7.58 (s, 1H), 7.54 (d, $J$ =5.6 Hz, 1H), 7.23-7.20 (dd, $J$ = 7.6, 5.2 Hz, 1H), 6.34 (s, 2H), 5.80 (d, $J$ =5.6 Hz, 1H), 3.97-3.94 (dd, $J$ = 11.6, 7.6 Hz, 2H), 3.39-3.37 (m, 2H), 2.03-1.94 (m, 2H), 1.64 (d, $J$ =13.6 Hz, 1H), 1.36 (d, $J$ =13.6 Hz, 1H).

In accordance with the synthesis method of Compound 10, Compounds 2-11 were synthesized from different Intermediates.

| Compound No. | Compound structure | Compound name | Intermediate | Analysis data [M+H]+ |
|---|---|---|---|---|
| Compound 2 | 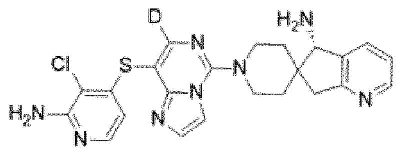 | (S)-1'-(8-((2-amino-3-chloropyridin-4-yl)thio)imidazo[1,2-c]pyrimidin-5-yl-7-deuterium)-5,7-dihydrospiro[cyclopentadieno[b]pyridine-6,4'-piperidine]-5-amine | A1, B2, C1 | 480.1 |
| Compound 3 | 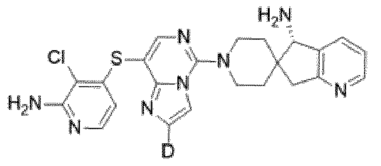 | (S)-1'-(8-((2-amino-3-chloropyridin-4-yl)thio)imidazo[1,2-c]pyrimidin-5-yl-2-deuterium)-5,7-dihydrospiro[cyclopentadieno[b]pyridine-6,4'-piperidine]-5-amine | A1, B3, C1 | 480.1 |
| Compound 4 | 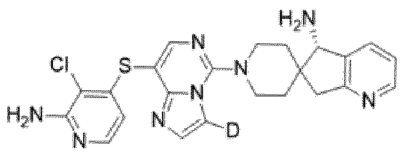 | (S)-1'-(8-((2-amino-3-chloropyridin-4-yl)thio)imidazo[1,2-c]pyrimidin-5-yl-3-deuterium)-5,7-dihydrospiro[cyclopentadieno[b]pyridine-6,4'-piperidine]-5-amine | A1, B4, C1 | 480.1 |
| Compound 5 | 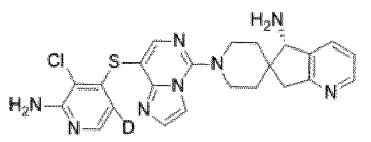 | (S)-1'-(8-((2-amino-3-chloropyridin-4-yl-5-deuterium)thio)imidazo[ 1,2-c]pyrimidin-5-yl)-5,7-dihydrospiro[cyclopentadieno[b]pyridine-6,4'-piperidine]-5-amine | A1, B1, C2 | 480.1 |
| Compound 6 | 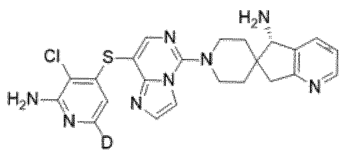 | (S)-1'-(8-((2-amino-3-chloropyridin-4-yl-6-deuterium)thio)imidazo[ 1,2-c]pyrimidin-5-yl)-5,7-dihydrospiro[cyclopentadieno[b]pyridine-6,4'-piperidine]-5-amine | A1, B1, C3 | 480.1 |
| Compound 7 | 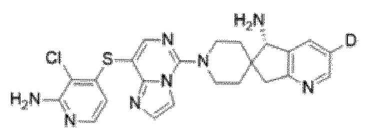 | (S)-1'-(8-((2-amino-3-chloropyridin-4-yl)thio)imidazo[1,2-c]pyrimidin-5-yl)-5,7-dihydrospiro[cyclopentadieno [b]pyridine-6,4'-piperidine]-3-deuterium-5-amine | A4, B1, C1 | 480.1 |
| Compound 8 | 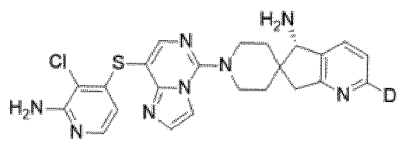 | (S)-1'-(8-((2-amino-3-chloropyridin-4-yl)thio)imidazo[1,2-c]pyrimidin-5-yl)-5,7-dihydrospiro[cyclopentadieno [b]pyridine-6,4'-piperidine]-2-deuterium-5-amine | A3, B1, C1 | 480.1 |
| Compound 9 | 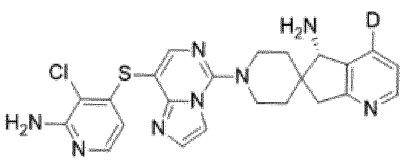 | (S)-1'-(8-((2-amino-3-chloropyridin-4-yl)thio)imidazo[1,2-c]pyrimidin-5-yl)-5,7-dihydrospiro[cyclopentadieno [b]pyridine-6,4'-piperidine]-4-deuterium-5-amine | A5, B1, C1 | 480.1 |
| | | | | |
| Compound 11 | 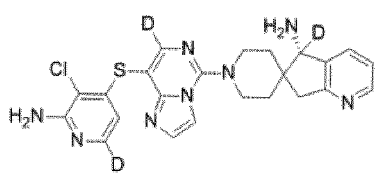 | (S)-1'-(8-((2-amino-3-chloropyridin-4-yl-6-deuterium)thio)imidazo[ 1,2-c]pyrimidin-5-yl)-5,7-dihydrospiro[cyclopentadieno[b]pyridine-6,4'-piperidine]-5-deuterium-5-amine | A2, B2, C3 | 482.1 |

Metabolic stability and pharmacokinetic data of the compounds in the present disclosure have been demonstrated in tests.

**Test Example 1: Liver microsomal metabolic stability test**

Experimental procedures:

1 Experimental method

1.1 Preparation of stock and working solutions

1) Preparation of stock and working solutions of the compound:
An appropriate amount of the compound was weighed and dissolved in DMSO to give a 10 mM stock solution, and the stock solution was further diluted with ACN to give a 100 μM working solution.
2) Preparation of stock and working solutions of the positive control
Each positive control standard was dissolved separately with DMSO to obtain a 10 mM stock solution and further diluted with ACN to obtain a 100 μM working solution.

1.2 Preparation of phosphate buffer solution
73.21 g of dipotassium hydrogen phosphate trihydrate and 10.78 g of potassium dihydrogen phosphate were dissolved in 4000 mL of ultrapure water. The pH of the solution was adjusted to 7.40 ± 0.10 with 10% phosphoric acid or 1 M potassium hydroxide and the final concentration was 100 mM.
1.3 Preparation of liver microsomal working solution (MWS)
Human liver microsomes (20 mg/mL) were diluted with the phosphate buffer solution to obtain 0.56 mg/mL of a liver microsomal working solution.
1.4 Preparation of cofactor (NADPH) solutions
NADPH was weighed and dissolved in the phosphate buffer solution to obtain a 10 mM solution.
1.5 Test steps

1) Preparation of Blank: 54 μL of human liver microsomal working solution was transferred to a blank sample plate, then added with 6 μL of NADPH working solution, and finally added with 180 μL of ACN solution containing 200 ng/mL of tolbutamide and labetalol (internal standard) to precipitate the protein.
2) Preparation of NADPH samples without cofactor (NCF60):

(1) 445 μL of human liver microsomal working solution (MWS) was added to the cofactor-free incubation sample plate, pre-incubated at 37 °C for 10 min, and then fully mixed after the addition of 5 μL of working solution of positive control or the compound to be tested.
(2) 50 μL of phosphate buffer solution was added to the above incubation sample plate, fully mixed and incubated at 37 °C for 60 min.
(3) After the incubation is complete, 60 μL of incubation solution was taken into a pellet plate, and then added with 180 μL of ACN solution containing 200 ng/mL of tolbutamide and labetalol (internal standard) to precipitate the protein.

3) Preparation of NADPH experimental research samples:

(1) 445 μL of human/rat liver microsomal working solution (MWS) was added to the incubation sample plate, pre-incubated at 37 °C for 10 min, and then fully mixed after the addition of 5 μL of working solution of positive control or the compound to be tested.
(2) 54 μL of the above intermediate working solution was transferred to the zero-time sample plate ($T_0$), then added with 180 μL of ACN solution containing 200 ng/mL tolubutamide and labetalol (internal standard) to precipitate the protein, and finally added with 6 μL of NADPH working solution.
(3) 44 μL of NADPH working solution was added to 396 μL of microsomal working solution containing positive control or the compound to be tested (remaining solution after the operation of steps (1) and (2)) and incubated at 37 °C. At the time points of 5, 15, 30, 45, and 60 min, 60 uL of incubation solution was taken and added to 180 μL of ACN solution containing 200 ng/mL of tolubutamide and labetalol (internal standard) to precipitate the protein.

1.6 Sample analysis

Centrifuged collected supernatants were analyzed with LC-MS/MS after appropriate dilution.

2. Data analysis

The peak area ratio of the analyte to the internal standard was used to calculate the relative percentage content of the compound after incubation and to perform exponential function fitting. The calculation formula was as follows:

1)

$$\text{Half-life } (T_{1/2}) = \text{Ln } (2)/k$$

wherein k was the rate constant obtained by linear regression taking the concentration logarithm as the ordinate and the incubation time as the absciss.

2)

$$\text{Intrinsic clearance } (CL_{int}) = \text{k/hepatic microsomal protein concentration}$$

wherein k was the rate constant obtained by linear regression taking the concentration logarithm as the ordinate and the incubation time as the absciss.

3)

$$\text{Hepatic clearance } (CL_{Hep}) = \text{Intrinsic clearance } (CL_{int}) \times \text{liver weight per kilogram of body weight} \times \text{hepatic microsomal proteins per gram of liver}$$

wherein $CL_{int}$ was the Intrinsic clearance. The results showed that the deuterated compound prepared by the present disclosure provided significantly improved metabolic stability in both human hepatic microsomes and rat hepatic microsomes as compared with its non-deuterated control compound.

| Compound No. | Human hepatic microsomal metabolic half-life (min) | Intrinsic clearance of human hepatic microsomes in body (μL/min/mg) |
|---|---|---|
| Compound 1 | 54 | 26 |
| Compound 10 | 72 | 19 |
| Compound 12 | 77 | 18 |

| Compound No. | Rat hepatic microsomal metabolic half-life (min) | Intrinsic clearance of rat hepatic microsomes in body (μL/min/mg) |
|---|---|---|
| Compound 1 | 70 | 19 |
| Compound 10 | 122 | 11.4 |

**Test example 2: Pharmacokinetics of compounds**

The following methods were used to determine the pharmacokinetic parameters of the compounds prepared in the present disclosure.

Healthy male adult mice/rats were used and each group of animals was administrated with 5-100mg of compounds of the present disclosure/Kg body weight by single gavage. Fasting was performed from 10 hours before administration to 4 hours after administration. Blood was collected after different time points after administration and the plasma content of the compound was determined (LC-MS/MS). Plasma concentration-time relationship was analyzed with specialized software (winnonlin) to calculate the pharmacokinetic parameters of each compound. The results showed that the deuterated compounds prepared by the present disclosure had a longer half-life, higher blood concentration

and better pharmacokinetic properties as compared with the non-deuterated control compounds.

All documents referred to in the present disclosure are cited in the present disclosure as a reference, as if each document were cited separately. Preferred embodiments of the present disclosure are described in detail. However, the present disclosure is not limited to the specific details of the above embodiments. Within the scope of the technical concept of the present disclosure, the technical solution of the present disclosure may be subjected to a variety of simple variants. These simple variants are within the scope of the protection of the present disclosure.

Further to be noted, the particular technical features described in the above specific embodiments, in the case of no contradiction, may be combined by any suitable manner. In order to avoid unnecessary repetition, the various possible combinations will not be described separately in the present disclosure. Further, various embodiments of the present disclosure may also be arbitrarily combined and should likewise be regarded as the content disclosed, as long as it does not violate the concept of the present disclosure.

## Claims

1. An isotope-substituted compound represented by formula I, or a pharmaceutically acceptable salt thereof, or an enantiomer, a diastereomer, a tautomer, a solvate, a polymorph, a prodrug, or a metabolite thereof:

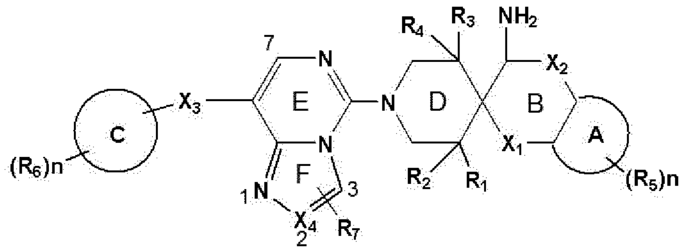

I

wherein,

$X_1$ and $X_2$ are each independently selected from a bond, O, $CR_aR_b$, or $NR_c$;
$X_3$ is selected from a bond, $CR_aR_b$, $NR_c$, S or O;
$X_4$ is selected from N or $CR_c$;
$R_a$, $R_b$ and $R_c$ are each independently selected from H, D, halogen, substituted or unsubstituted $C_{1-6}$ alkyl, or substituted or unsubstituted $C_{1-6}$ alkoxyl;
$R_1$, $R_2$, $R_3$, $R_4$ and $R_7$ are each independently selected from H, D, -OH, halogen, substituted or unsubstituted amino, substituted or unsubstituted $C_{1-6}$ alkyl, or substituted or unsubstituted $C_{1-6}$ alkoxyl; and $R_1$ and $R_2$ are not -OH or $-NH_2$ at the same time, $R_3$ and $R_4$ are not -OH or $-NH_2$ at the same time;
ring A is selected from substituted or unsubstituted $C_{4-8}$ cyclic hydrocarbyl hydrocarbyl, substituted or unsubstituted 4-8 membered heterocyclyl, substituted or unsubstituted $C_{5-10}$ aryl groups, or substituted or unsubstituted 5-10 membered heteroaryl, wherein the heterocyclyl or heteroaryl comprises 1-3 heteroatoms selected from the group consisting of N, O, S and P;
ring C is selected from substituted or unsubstituted $C_{4-8}$ cyclic hydrocarbyl, substituted or unsubstituted 5-6 membered monocyclic heterocyclyl, substituted or unsubstituted 8-10 membered bicyclic heterocyclyl, substituted or unsubstituted $C_{5-10}$ monocyclic or bicyclic aryl, substituted or unsubstituted 5-6 membered monocyclic heteroaryl, or substituted or unsubstituted 8-10 membered bicyclic heteroaryl, wherein the heterocyclyl or heteroaryl comprise 1-4 heteroatoms selected from the group consisting of N, O, S and P;
$R_5$ and $R_6$ are each independently selected from H, D, -OH, halogen, cyano, $-NO_2$, substituted or unsubstituted amino, substituted or unsubstituted $C_{1-6}$ alkyl, or substituted or unsubstituted $C_{1-6}$ alkoxyl;
n is any integer from 0 to 3; and
wherein the substitution refers to one or more hydrogen atoms on the group is substituted by a substituent selected from the group consisting of halogen, -OH, $-NO_2$, $-NH_2$, -NH (unsubstituted or halogenated $C_{1-6}$ alkyl), -N(unsubstituted or halogenated $C_{1-6}$ alkyl)$_2$, -CN, unsubstituted or halogenated $C_{1-8}$ alkyl, unsubstituted or halogenated $C_{1-8}$ alkoxyl, unsubstituted or halogenated $C_{1-8}$ alkoxyl-$C_{1-8}$ alkyl, unsubstituted or halogenated $C_{3-8}$ cycloalkyl-$C_{1-8}$ alkyl, unsubstituted or halogenated $C_{1-6}$ alkylcarbonyl, unsubstituted or halogenated $C_{1-6}$ alkoxylcarbonyl, isohydroxamic acid group, unsubstituted or halogenated $C_{1-6}$ alkyl mercapto, $-S(O)_2N$ (unsubstituted or halogenated $C_{1-6}$ alkyl)$_2$, $-S(O)_2$ unsubstituted or halogenated $C_{1-6}$ alkyl, -N(unsubstituted or halo-

genated $C_{1-6}$ alkyl)$S(O)_2N$(unsubstituted or halogenated $C_{1-6}$ alkyl)$_2$, -S(O)N(unsubstituted or halogenated $C_{1-6}$ alkyl)$_2$, -S(O)(unsubstituted or halogenated $C_{1-6}$ alkyl), -N(unsubstituted or halogenated $C_{1-6}$ alkyl)S(O)N(unsubstituted or halogenated $C_{1-6}$ alkyl)$_2$, -N(unsubstituted or halogenated $C_{1-6}$ alkyl)S(O)(unsubstituted or halogenated $C_{1-6}$ alkyl), unsubstituted or halogenated $C_{5-10}$ aryl, unsubstituted or halogenated 5-10 membered heteroaryl, unsubstituted or halogenated $C_{4-8}$ cyclic hydrocarbyl, and unsubstituted or halogenated 4-8 membered heterocyclyl, wherein the heterocyclyl and the heteroaryl comprise 1-4 heteroatoms selected from the group consisting of N, O and S;
wherein the isotope-substitution refers to one or more ring carbon atoms in one or more rings of ring A, ring B, ring C, ring D, ring E and ring F are substituted with $^{13}C$, and/or hydrogen atoms on one or more ring atoms in one or more rings of ring A, ring B, ring C, ring D, ring E and ring F are substituted with deuterium.

2. The isotope-substituted compound represented by formula I according to claim 1, wherein isotope-substitution is deuterated, wherein

hydrogen atoms on one or more ring atoms in ring A, ring B, ring C, ring E and ring F are substituted with deuterium; preferably, in the isotope-substituted compound represented by formula I, deuterium-substitution occurs in ring B, i.e., hydrogen atoms on one or more ring atoms of ring B are substituted with deuterium; or
wherein hydrogen atoms at one or more positions selected from the following positions are deuterated: hydrogen atoms on the ring atom at any positions other than heterocyclic atoms in ring A; and/or, hydrogen atoms on the ring atoms substituted with amino in ring B, and/or, hydrogen atoms on $X_1$ and/or $X_2$; and/or, hydrogen atoms on the ring atom at any positions other than heterocyclic atom in ring C; and/or, hydrogen atoms on the ring atom at 7-position of ring E; and/or, hydrogen atoms on the ring atoms at 2- and 3-positions of ring F; preferably, the total number of hydrogen atoms that are deuterated in the compound of formula I is 1 to 4.

3. The isotope-substituted compound represented by formula I according to claim 1 or 2, wherein ring C is:

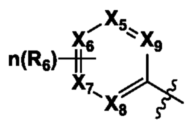,
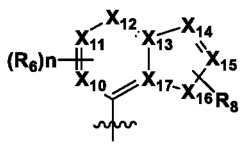
or
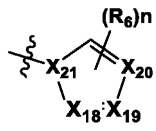;

wherein,

$X_5$, $X_6$, $X_7$, $X_8$ and $X_9$ are each independently selected from N or $CR_d$; and at most three of them are N at the same time;
$X_{10}$, $X_{11}$, $X_{12}$, $X_{13}$, $X_{14}$, $X_{15}$, $X_{16}$ and $X_{17}$ are each independently selected from N or $CR_d$; and at most five of them are N at the same time;
$X_{18}$, $X_{19}$, $X_{20}$ and $X_{21}$ are each independently selected from N or $CR_d$; and at most three of them are N at the same time;
$R_6$ and $R_8$ are each independently selected from H, D, $-NH_2$, -CN, -OH, -NOz, halogen, unsubstituted or halogenated $C_{1-6}$ alkyl, or unsubstituted or halogenated $C_{1-6}$ alkoxyl; and
$R_d$ is selected from H, D, halogen, unsubstituted or halogenated $C_{1-6}$ alkyl, or unsubstituted or halogenated $C_{1-6}$ alkoxyl;
wherein the wavy line indicates the position where ring C and $X_3$ are connected.

4. The isotope-substituted compound represented by formula I according to claim 3, wherein ring C is

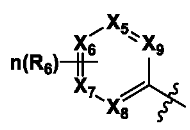
or
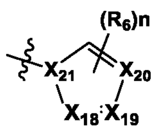,

wherein,

0, 1 or 2 of $X_5$, $X_6$, $X_7$, $X_8$ and $X_9$ are N and the rest are $CR_d$;
0, 1 or 2 of $X_{18}$, $X_{19}$, $X_{20}$ and $X_{21}$ are N and the rest are $CR_d$;

$R_6$ is selected from H, D, $-NH_2$, -CN, -OH, $-NO_2$, -F, -Cl, -Br, methyl, ethyl, propyl, isopropyl, butyl, methoxy, ethoxy, propoxy, isopropoxy, fluorinated or brominated $C_{1-3}$ alkyl, or fluorinated or brominated $C_{1-3}$ alkoxyl; and said $R_d$ is selected from H, D, -F, -Cl, -Br, methyl, ethyl, propyl, isopropyl, butyl, methoxy, ethoxy, propoxy, isopropoxy, fluorinated or brominated $C_{1-3}$ alkyl, or fluorinated or brominated $C_{1-3}$ alkoxyl;
wherein the wavy line indicates the position where ring C and $X_3$ are connected.

**5.** The isotope-substituted compound represented by formula I according to claim 3, wherein ring C is:

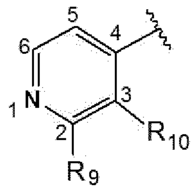

;

wherein $R_9$ and $R_{10}$ are each independently H, halogen, -NR'R" or unsubstituted $C_{1-6}$ alkyl groups, where R' and R" are each independently H or $C_{1-4}$ alkyl; preferably, $R_9$ is -NR'R", $R_{10}$ is halogen;
preferably, ring C is:

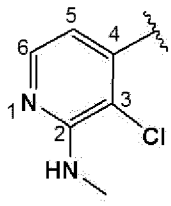 , 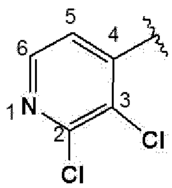 or 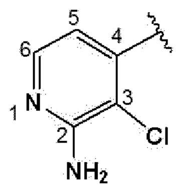 ;

preferably, hydrogen atoms at 5- and/or 6- positions of the pyridine ring are substituted with deuterium;
more preferably, said ring C is:

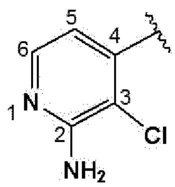

wherein hydrogen atoms at 5- and/or 6- positions are not substituted with deuterium, or 1-3 hydrogen atoms at 5- and/or 6- positions are substituted with deuterium.

**6.** The isotope-substituted compound represented by formula I according to any one of claims 1-5, wherein ring A is selected from substituted or unsubstituted $C_{4-6}$ cyclic hydrocarbyl, substituted or unsubstituted 4-6 membered heterocyclyl, substituted or unsubstituted $C_{5-6}$ aryl, or substituted or unsubstituted 5-6 membered heteroaryl, wherein the heterocyclyl or heteroaryl comprise 1-3 N atoms;
preferably, ring A is:

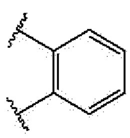 , 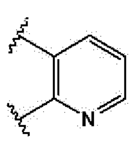 , 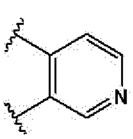 , 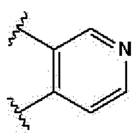 , 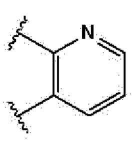 , 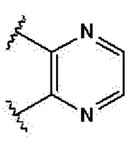 ,

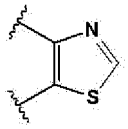, 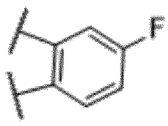, 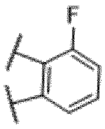, or 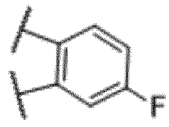,

preferably

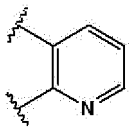;

wherein the wavy line represents the position of ring A fused with ring B, preferably, one or more hydrogen atoms at one or more ring atom positions other than heteroatoms and the ring atom positions that are substituted with F in ring A are substituted with deuterium.

**7.** The isotope-substituted compound represented by formula I according to claim 6, wherein ring A is any one selected from the group consisiting of:

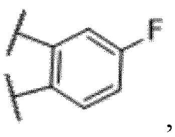, 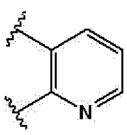, 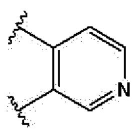, 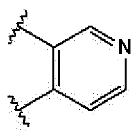, 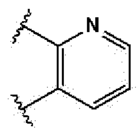, and 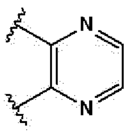;

wherein, one or more hydrogen atoms at the ring atom positions other than the ring nitrogen atom and the ring atom positions that are substituted with F in the ring A are substituted with D;
preferably, said ring A is:

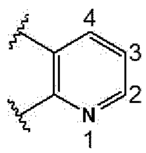,

wherein the isotope-substitution is deuterated at 2-, 3- and/or 4-positions.

**8.** The isotope-substituted compound represented by formula I according to any one of claims 1-7, wherein,

$R_1$, $R_2$, $R_3$, $R_4$ and $R_7$ are each independently selected from H, D, -OH, -F, -Cl, -Br,-$NH_2$, -$NHC_{1-3}$ alkyl, methyl, ethyl, propyl, isopropyl, butyl, methoxy, ethoxy, propoxy, isopropoxy, $C_{1-3}$ alkyl that is substituted with halogen, -$NH_2$, -OH, $C_{1-3}$ alkyl or $C_{1-3}$ alkoxyl, or $C_{1-3}$ alkoxyl that is substituted with halogen, -$NH_2$, -OH, $C_{1-3}$ alkyl or $C_{1-3}$ alkoxyl; and $R_1$ and $R_2$ are not -OH or -$NH_2$ at the same time, and $R_3$ and $R_4$ are not -OH or -$NH_2$ at the same time; and/or
$R_5$ and $R_6$ are each independently selected from H, D, -OH, -F, -Cl, -Br, -CN, -$NH_2$, -$NHC_{1-3}$ alkyl, methyl, ethyl, propyl, isopropyl, butyl, methoxy, ethoxy, propoxy, isopropoxy, $C_{1-3}$ alkyl that is substituted with halogen, -$NH_2$, -OH, $C_{1-3}$ alkyl or $C_{1-3}$ alkoxyl, or $C_{1-3}$ alkoxyl that is substituted with halogen, -$NH_2$, -OH, $C_{1-3}$ alkyl or $C_{1-3}$ alkoxyl; and/or
the substituent is selected from -F, -Cl, -Br, -OH, -NOz, -$NH_2$, -NH($C_{1-6}$ alkyl), -N($C_{1-6}$ alkyl)$_2$, -CN, $C_{1-6}$ alkyl, $C_{1-4}$ alkoxyl, $C_{1-4}$ alkoxyl-$C_{1-6}$ alkyl, $C_{3-8}$ cycloalkyl-$C_{1-8}$ alkyl, $C_{1-6}$ alkyl carbonyl, $C_{1-6}$ alkoxylcarbonyl, $C_{1-6}$ alkyl mercapto, -$S(O)_2N$($C_{1-6}$ alkyl)$_2$, -$S(O)_2C_{1-6}$ alkyl, -N($C_{1-6}$ alkyl)$S(O)_2N$($C_{1-6}$ alkyl)$_2$, -S(O)N($C_{1-6}$ alkyl)$_2$, -S(O)($C_{1-6}$ alkyl), -N($C_{1-6}$ alkyl)S(O)N($C_{1-6}$ alkyl)$_2$, -N($C_{1-6}$ alkyl)S(O)($C_{1-6}$ alkyl), substituted or unsubstituted $C_{5-10}$ aryl, substituted or unsubstituted 5-10 membered heteroaryl, substituted or unsubstituted $C_{4-8}$ cyclic hydrocarbyl, or substituted or unsubstituted 4-8 membered heterocyclyl, wherein the heterocyclyl and heteroaryl

comprise 1-4 heteroatoms selected from the group consisting of N, O and S.

**9.** The isotope-substituted compound represented by formula I according to any one of claims 1-7, wherein,

$R_1$, $R_2$, $R_3$, $R_4$ and $R_7$ are each independently selected from the group consisting of H, D and $C_{1-3}$ alkyl;
each $R_5$ is independently selected from the group consisting of H, D and $C_{1-3}$ alkyl;
each $R_6$ is independently selected from the group consisting of H, D, $C_{1-3}$ alkyl, halogen and amino, preferably selected from the group consisting of H, D, halogen and amino.

**10.** The isotope-substituted compound represented by formula I according to claim 8 or 9, wherein,

$X_1$ and $X_2$ are each independently selected from a bond or $CR_aR_b$;
$X_3$ is selected from S or O;
$X_4$ is $CR_c$;
$R_a$, $R_b$ and $R_c$are each independently selected from the group consisting of H, D and $C_{1-3}$ alkyl;
n is any integer from 0 to 2.

**11.** The isotope-substituted compound represented by formula I according to claim 1 or 2, wherein ring C is

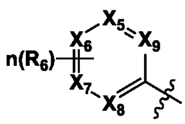,
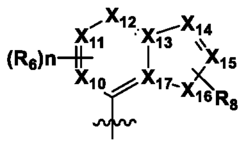
or
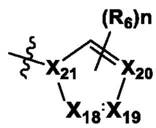;

wherein,

$X_5$, $X_6$, $X_7$, $X_8$ and $X_9$ are each independently selected from N or $CR_d$; and at most three of them are N at the same time;
$X_{10}$, $X_{11}$, $X_{12}$, $X_{13}$, $X_{14}$, $X_{15}$, $X_{16}$ and $X_{17}$ are each independently selected from N or $CR_d$; and at most five of them are N at the same time;
$X_{18}$, $X_{19}$, $X_{20}$ and $X_{21}$ are each independently selected from N or $CR_d$; and at most three of them are N at the same time;
$R_6$ and $R_8$ are each independently selected from H, $-NH_2$, -CN, -OH, -NOz, halogen, unsubstituted or halogenated $C_{1-6}$ alkyl, or unsubstituted or halogenated $C_{1-6}$ alkoxyl; and
$R_d$ is selected from H, halogen, unsubstituted or halogenated $C_{1-6}$ alkyl, or unsubstituted or halogenated $C_{1-6}$ alkoxyl;
wherein the wavy line indicates the position where ring C and $X_3$ are connected.

**12.** The isotope-substituted compound represented by formula I according to claim 11, wherein said ring C is

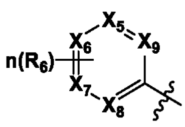
or
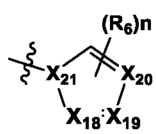

wherein,

0, 1 or 2 of $X_5$, $X_6$, $X_7$, $X_8$ and $X_9$ are N and the rest are $CR_d$;
0, 1 or 2 of $X_{18}$, $X_{19}$, $X_{20}$ and $X_{21}$ are N and the rest are $CR_d$;
$R_6$ is selected from H, $-NH_2$, -CN, -OH, -NOz, -F, -Cl, -Br, methyl, ethyl, propyl, isopropyl, butyl, methoxy, ethoxy, propoxy, isopropoxy, fluorinated or brominated $C_{1-3}$ alkyl, or fluorinated or brominated $C_{1-3}$ alkoxyl; and
said $R_d$ is selected from H, -F, -Cl, -Br, methyl, ethyl, propyl, isopropyl, butyl, methoxy, ethoxy, propoxy, isopropoxy, fluorinated or brominated $C_{1-3}$ alkyl, or fluorinated or brominated $C_{1-3}$ alkoxyl;
wherein the wavy line indicates the position where ring C and $X_3$ are connected.

**13.** The isotope-substituted compound represented by formula I according to any one of claims 1-7, wherein,

$R_1$, $R_2$, $R_3$, $R_4$ and $R_7$ are each independently selected from H, -OH, halogen, substituted or unsubstituted amino, substituted or unsubstituted $C_{1-6}$ alkyl, or substituted or unsubstituted $C_{1-6}$ alkoxyl; and $R_1$ and $R_2$ are not -OH or -$NH_2$ at the same time, $R_3$ and $R_4$ are not -OH or -$NH_2$ at the same time; and/or
$X_3$ is selected from $CR_aR_b$, $NR_c$, S or O; and/or
ring A is selected from substituted or unsubstituted $C_{4-8}$ cyclic hydrocarbyl, substituted or unsubstituted 4-8 membered heterocyclyl, substituted or unsubstituted $C_{5-10}$ aryl groups, or substituted or unsubstituted 5-10 membered heteroaryl, wherein the heterocyclyl or heteroaryl comprises 1-3 heteroatoms selected from the group consisting of N, O, S and P; and/or
ring C is selected from substituted or unsubstituted $C_{4-8}$ cyclic hydrocarbyl, substituted or unsubstituted 5-6 membered monocyclic heterocyclyl, substituted or unsubstituted 8-10 membered bicyclic heterocyclyl, substituted or unsubstituted C5-10 bicyclic aryl, substituted or unsubstituted 5-6 membered monocyclic heteroaryl, or substituted or unsubstituted 8-10 membered bicyclic heteroaryl, wherein the heterocyclyl or heteroaryl comprise 1-4 heteroatoms selected from the group consisting of N, O, S and P;
$R_5$ and $R_6$ are each independently selected from H, -OH, halogen, cyano, -NOz, unsubstituted amino, unsubstituted $C_{1-6}$ alkyl, or unsubstituted $C_{1-6}$ alkoxyl.

**14.** The isotope-substituted compound represented by formula I according to claim 1, wherein, ring A is:

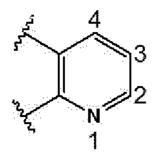

,

wherein the wavy line represents the position of ring A fused with ring B;
in ring B, one of $X_1$ and $X_2$ is a bond, and the other is $CH_2$;
$X_3$ is S or O, preferably S;
$X_4$ is CH;
$R_1$ and $R_3$ are each independently H and unsubstituted $C_{1-6}$ alkyl, preferably both are H;
$R_2$ and $R_4$ are each independently H and unsubstituted $C_{1-6}$ alkyl, preferably both are H;
$R_7$ is H, hydroxyl, halogen and unsubstituted $C_{1-6}$ alkyl, preferably H or $C_{1-4}$ alkyl;
ring C is:

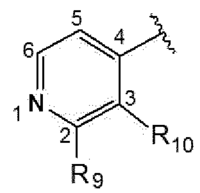

,

wherein $R_9$ and $R_{10}$ are each independently H, halogen, amino or unsubstituted $C_{1-6}$ alkyl, preferably are each independently halogen, amino or $C_{1-6}$ alkyl; more preferably, $R_9$ and $R_{10}$ are both halogens, or one of $R_9$ and $R_{10}$ is halogen and the other is $C_{1-6}$ alkyl, or $R_9$ is amino, $R_{10}$ is halogen; where the wavy line represents the position where ring C is connected to $X_3$;
wherein the isotope substation refers to 1-4 hydrogen atoms on the ring atom are replaced by deuterium, wherein the hydrogen atoms that are substituted with deuterium are selected from one or more of the following positions:
hydrogen atoms at 2-, 3-, 7- positions in imidazo[1,2-c]pyrimidine ring; and/or
hydrogen atoms at 5-, 6- positions when ring C is a pyridine ring, hydrogen atoms at 4-, 5-, 6-positions when ring C is a benzene ring; and/or
hydrogen atoms at 2-, 3-, 4- positions in ring A; and/or
hydrogen atoms on the carbon atoms substituted with amino in ring B, and/or hydrogen atoms attached to $X_1$ and $X_2$ when they are not a bond; preferably hydrogen atoms on the carbon atoms substituted with amino in ring B, or one or two of one or two hydrogen atoms attached to $X_1$ and $X_2$ when they are not a bond and hydrogen

atoms on the carbon atoms substituted with amino.

**15.** The isotope-substituted compound represented by formula I according to claim 1 selected from:

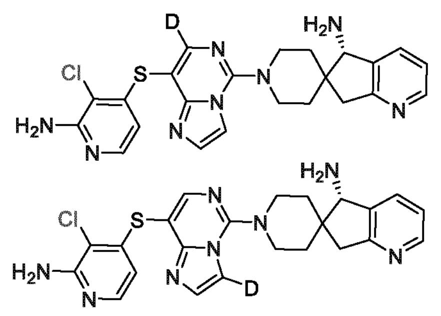

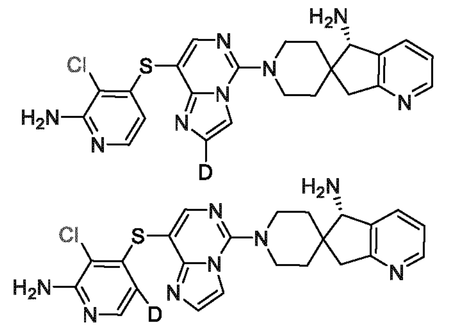

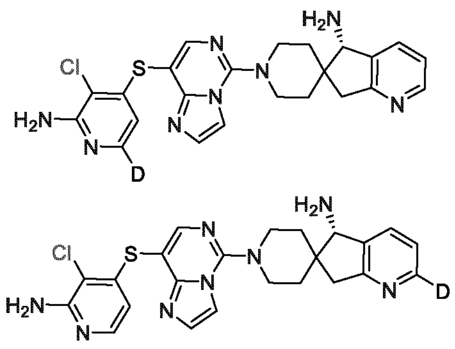

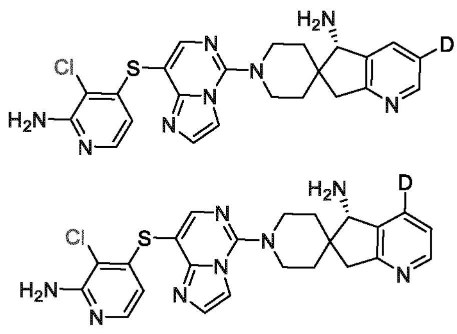

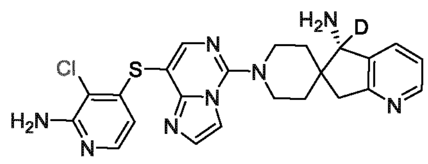

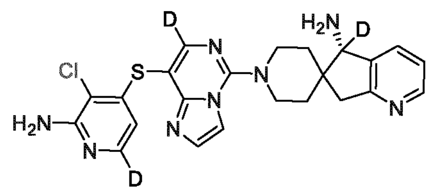

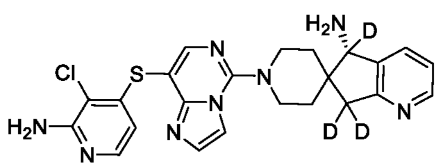
,

and a pharmaceutically acceptable salt thereof, an enantiomer, a diastereomer, a tautomer, a solvate, a polymorph, a prodrug, and a metabolite thereof.

**16.** The isotope-substituted compound represented by formula I according to claim 1 selected from

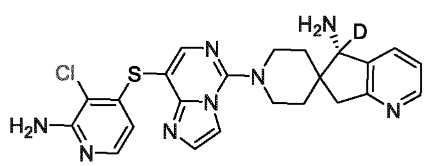

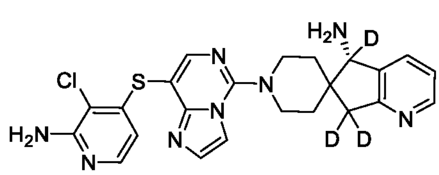
;

and a pharmaceutically acceptable salt thereof, an enantiomer, a diastereomer, a tautomer, a solvate, a polymorph, a prodrug, and a metabolite thereof.

**17.** A method for preparing the isotope-substituted compound represented by formula I according to any one of claims 1-16, wherein the method comprises the following steps: reacting a compound of formula Ib with a compound of formula Ic by a nucleophilic substitution to obtain a compound of formula Id;

reacting the compound of formula Id with a compound of formula Ie by substitution to obtain a compound of formula If; and
deprotecting the compound of formula If with an acid to obtain a compound of formula I:

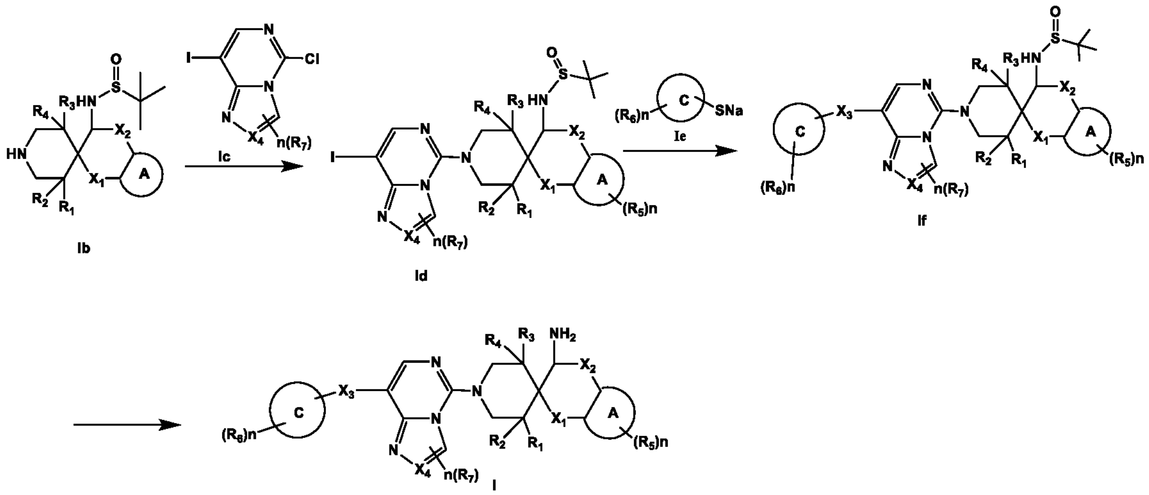


wherein, said $R_1$-$R_4$, n, ring A, $X_1$, $X_2$, $X_3$, $X_4$ and ring C are as defined in any one of claims 1-16.

**18.** The method according to claim 17, wherein the compound of formula Ib is a compound represented by the following formula IIc, which is prepared by using a method comprising the following steps:

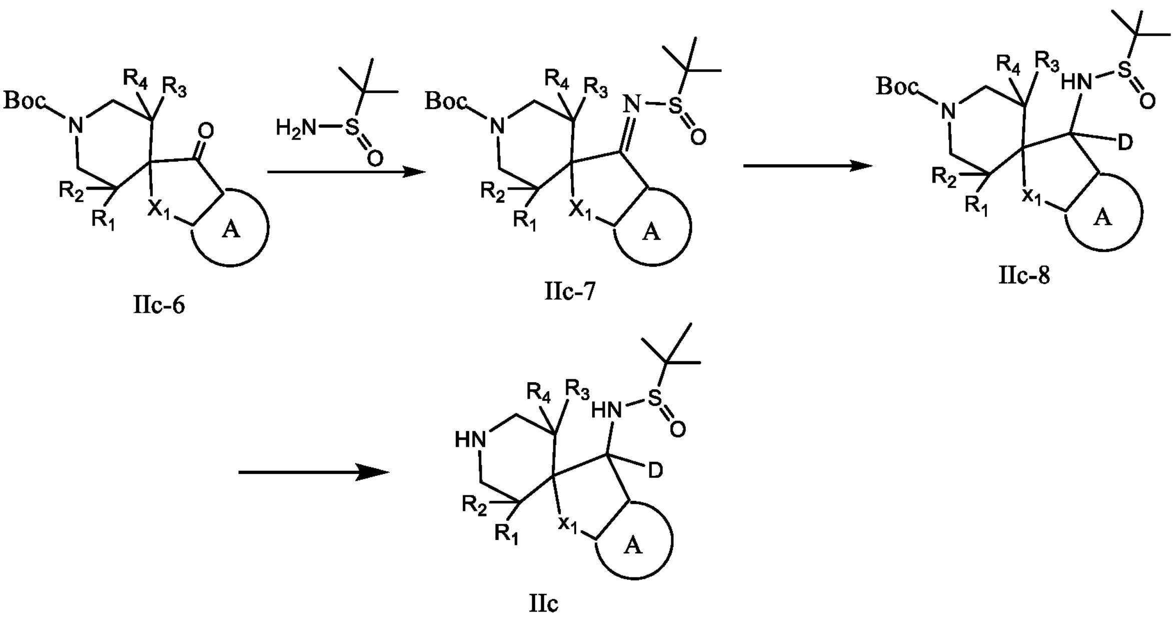


(1) a compound of formula IIc-6 is reacted with chiral tert-butyl sulfinamide in a solvent to obtain a compound of formula IIc-7; wherein the solvent is preferably an organotitanate compound, more preferably tetraisopropyl titanate;
(2) the compound of formula IIc-7 is reduced to a compound of formula IIc-8 by a deuterated reducing agent in a deuterated alcohol solvent; wherein the deuterated alcohol solvent is preferably deuterated methanol; and the deuterated reducing agent is preferably an alkali metal borodeuteride, more preferably potassium borodeuteride, sodium borodeuteride, most preferably sodium borodeuteride;

(3) the protective groups of the compound of formula IIc-8 is deprotected under the action of organic acids and haloalkanes to obtain the compound of formula IIc; wherein the organic acid is preferably haloalkyl acid, more preferably haloacetic acid, further preferably trifluoroacetic acid; the haloalkanes are preferably chlorinated alkanes, more preferably dichloromethane, carbon tetrachloride, further preferably dichloromethane;
wherein said ring A, $R_1$-$R_4$ and $X_1$ in formula IIc-6, IIc-7, IIc-8 and IIc are as defined in any one of claims 1-16.

**19.** The method according to claim 18, wherein the compound of formula IIc-6 is a compound represented by the following formula IIa-6, which is prepared by using a method comprising the following steps:

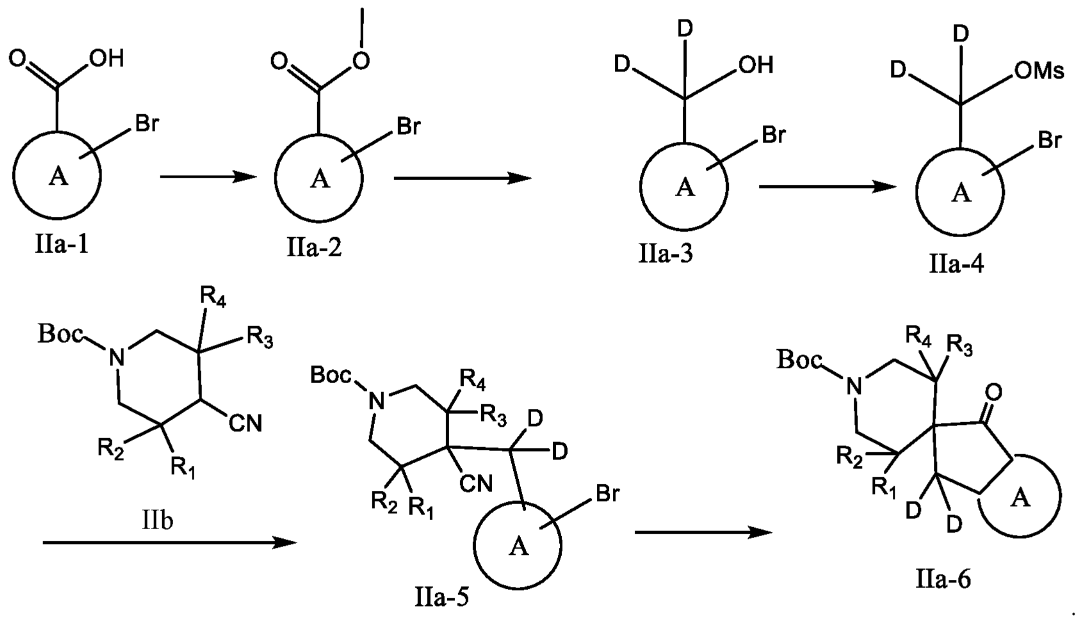
IIa-1 IIa-2 IIa-3 IIa-4 IIb IIa-5 IIa-6

;

(i) a compound of formula IIa-1 and alcohol solvents undergo esterification reaction under the catalysis of thionyl chloride to obtain a compound of IIa-2; wherein the alcohol solvent is preferably a fatty alcohol, more preferably methanol, ethanol, propanol, most preferably methanol;
(ii) the compound of formula IIa-2 is reduced to a compound of formula IIa-3 by the deuterated reducing agent in a deuterated alcohol solvent; wherein the deuterated alcohol solvent is preferably deuterated alkyl alcohol, further preferably deuterated methanol, deuterated ethanol, most preferably deuterated methanol; and the deuterated reducing agent is preferably an alkali metal borodeuteride, more preferably potassium borodeuteride, sodium borodeuteride, most preferably sodium borodeuteride;
(iii) the compound of formula IIa-3 is substituted with a methylsulfonyl group in the presence of an organic solvent and an organic base to obtain a compound of formula IIa-4; wherein the organic solvent is preferably a haloalkane, more preferably dichloromethane; and the organic base is preferably an alkyl amine, more preferably C1-3 alkyl amine, triethylamine;
(iv) in the presence of organic solvents and metal organic bases, the compound of formula IIa-4 is reacted with a compound of formula lib to obtain a compound of formula IIa-5; wherein the metal organic base is preferably LDA; and the organic solvent is preferably an ether solvent, more preferably THF; and
(v) the compound of formula IIa-5 is catalyzed by a palladium catalyst in the presence of an organic solvent and an organic base, and undergoes cyclization under heating to obtain a compound of formula IIa-6; wherein the palladium catalyst is preferably Pd(aMphos)Clz, the solvent is preferably DMA/$H_2O$, and the organic base is preferably triethylamine;

wherein ring A in formulae IIa-1, IIa-2, IIa-3, IIa-4, IIa-5 and IIa-6 and $R_1$-$R_4$ in formulae IIb, IIa-5 and IIa-6 are each as defined in any one of claims 1-16.

**20.** Use of the isotope-substituted compound represented by formula I according to any one of claims 1-16, or a pharmaceutically acceptable salt thereof, or an enantiomer, a diastereomer, a tautomer, a solvate, a polymorph, a prodrug, or a metabolite thereof in the following methods:

(a) preparing drugs for the prevention or treatment of diseases or disorders associated with abnormal activity/level of SHP2;
(b) preparing drugs for the prevention or treatment of SHP2-mediated diseases or disorders;
(c) preparing inhibitor drugs that inhibit SHP2 activity/level;
(d) non-therapeutically inhibiting SHP2 activity/level in vitro;
(e) non-therapeutically inhibiting tumor cell proliferation in vitro; or
(f) treating diseases or conditions associated with abnormal SHP2 activity/level.

**21.** Use according to claim 20, wherein the disease is a cancer, including but not limited to Noonan syndrome, Leopard syndrome, adolescent myelomonocytic leukemia, neuroblastoma, melanoma, acute myeloid leukemia, breast cancer, esophageal cancer, lung cancer, colon cancer, head cancer, neuroblastoma, squamous cell carcinoma of the head and neck, gastric cancer, anaplastic large cell lymphoma, glioblastoma, hepatocellular carcinoma (HCC), acute lymphoblastic leukemia, adrenal cortex carcinoma, anal cancer, appendix cancer, astrocytomas, atypical malformations/tumoroids, basal cell carcinoma, cholangiocarcinoma, bladder cancer, bone cancer (osteosarcoma and malignant fibrous histiocytoma), brainstem glioma, brain tumor, brain and spinal cord tumor, bronchial tumor, Burkitt lymphoma, cervical cancer, chronic lymphocytic leukemia, chronic myelogenous leukemia, colorectal cancer, craniopharyngioma, embryonic tumor, endometrial cancer, epithelial cell tumors, ependymomas, Ewing sarcoma family tumors, eye cancer, retinoblastoma, gallbladder carcinoma, gastrointestinal carcinoid, gastrointestinal stromal tumors (GIST), gastrointestinal stromal cell tumors, germ cell tumors, gliomas, hair cell leukemia, head and neck cancer, Hodgkin lymphoma, hypopharyngeal cancer, islet cell tumor (endocrine pancreas), Kapozi sarcoma, kidney cancer, Langerhans cell histiocytosis, laryngeal cancer, leukemia, hair cell leukemia, liver cancer, non-small cell lung cancer, small cell lung cancer, lymphoma, medulloblastoma, medullary epithelioma, mesothelioma, oral cancer, multiple myeloma, nasopharyngeal cancer, neuroblastoma, non-Hodgkin lymphoma, oropharyngeal cancer, osteosarcoma, malignant bone fibrous histiocytoma, ovarian cancer, ovarian epithelial carcinoma, ovarian germ cell tumor, ovarian low malignant potential tumor, pancreatic cancer, papillomatosis, parathyroid carcinoma, penile cancer, pharyngeal cancer, pineal intermediate differentiation tumor, osteoblastoma and supratentorial primitive neuroectodermal tumor, pituitary tumor, plasma cell tumor/multiple myeloma, pleural pneumocytoma, primary central nervous system lymphoma, prostate cancer, rectal cancer, kidney cell (kidney) cancer, retinoblastoma, rhabdomyosarcoma, salivary adenocarcinoma, sarcoma, Ewing sarcoma family tumors, sarcoma, Kaposi disease, Sezary syndrome, skin cancer, small intestinal carcinoma, soft tissue sarcoma, squamous cell carcinoma, supratentorial primitive neuroectodermal tumor, T-cell lymphoma, testicular cancer, laryngeal cancer, thymoma and thymus cancer, thyroid cancer, urethral cancer, uterine cancer, uterine sarcoma, vaginal cancer, vulvar cancer, Waldenstrom macroglobulinemia and Wilms tumors.

**22.** A pharmaceutical composition comprising:

(i) an effective amount of the isotope-substituted compound represented by formula I of any one of claims 1-16, or a pharmaceutically acceptable salt, an enantiomer, a diastereomer, a tautomer, a solvate, a polymorph, a prodrug or a metabolite thereof; and
(ii) a pharmaceutically acceptable carrier.

**23.** The pharmaceutical composition according to claim 22 further comprising other therapeutic agents.

**24.** A method of preparing a pharmaceutical composition, which comprises mixing an isotope substituted compound represented by formula I according to any one of claims 1-16 or a pharmaceutically acceptable salt thereof, or an enantiomer, a diastereomer, a tautomer, a solvate, a polymorph, a prodrug or a metabolite thereof with a pharmaceutically acceptable carrier.

**INTERNATIONAL SEARCH REPORT**

International application No. **PCT/CN2021/098604**

**A. CLASSIFICATION OF SUBJECT MATTER**

C07D 401/04(2006.01)i; C07D 401/14(2006.01)i; C07D 471/10(2006.01)i; C07D 487/04(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61P

Documentation searched other than minimum documentation to the extent that such documents are included in the fields searched

Electronic data base consulted during the international search (name of data base and, where practicable, search terms used)

DWPI; CNABS; SIPOABS; CNTXT; WOTXT; USTXT; EPTXT; Web of science; CNKI; Patentics; 万方; STN-REGISTRY; STN-CAPLUS: 上海奕拓医药, SHP2, 嘧啶, 咪唑, 三氮唑, 哌啶, 螺, 结构式I, structural I, +pyrimind+, +piperidin+, +spir+, +imidaz+, cancer, tumor, 癌, 肿瘤

**C. DOCUMENTS CONSIDERED TO BE RELEVANT**

| Category* | Citation of document, with indication, where appropriate, of the relevant passages | Relevant to claim No. |
|---|---|---|
| X | WO 2020063760 A1 (JACOBIO PHARMACEUTICALS CO., LTD.) 02 April 2020 (2020-04-02)<br>page 96, table, compound 22, pages 88, 89 and 92, and claims 1, 58 and 77 | 1-18, 20-24 |
| X | WO 2020108590 A1 (TUOJIE BIOTECH (SHANGHAI) CO., LTD.) 04 June 2020 (2020-06-04)<br>pages 57-60, embodiment 27, and claim 1 | 1-18, 20-24 |
| X | CN 111138412 A (SHANGHAI YITUO PHARMACEUTICAL TECHNOLOGY CO., LTD.) 12 May 2020 (2020-05-12)<br>description, paragraphs [0292]-[0301] and [0495]-[0501], and claims 1 and 7 | 1-24 |
| A | CN 111153901 A (RUDONG LINGDA BIOMEDICAL TECH CO., LTD.) 15 May 2020 (2020-05-15)<br>claims 1-20 and 23-28 | 1-24 |
| A | WO 2020072656 A1 (GILEAD SCIENCES, INC.) 09 April 2020 (2020-04-09)<br>claims 1, 68 and 69 | 1-24 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 July 2021** | **23 August 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/098604**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20、21**
because they relate to subject matter not required to be searched by this Authority, namely:

[1] Claims 20 and 21 relate to a treatment method of mammal for BET protein-mediated disease, the implementation objects of which are the living human or animal body, the direct purpose of which is curing disease, which is a method for treatment of the human or animal body, and therefore does not warrant an international search according to the criteria set out in PCT Rule 39.1(iv).

[2] The search for claims 20 and 21 is made on the basis of reasonable expectations made the following amendments: scheme (f) of claim 20 was amended to "preparation of drugs for the treatment of diseases or disorders related to abnormal SHP2 activity/level".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/098604**

| Patent document cited in search report | Publication date (day/month/year) | Patent family member(s) | Publication date (day/month/year) |
|---|---|---|---|
| WO 2020063760 A1 | 02 April 2020 | None | |
| WO 2020108590 A1 | 04 June 2020 | CA 3120791 A1 | 04 June 2020 |
| | | AU 2019386036 A1 | 27 May 2021 |
| | | TW 202039498 A | 01 November 2020 |
| CN 111138412 A | 12 May 2020 | WO 2020094018 A1 | 14 May 2020 |
| | | CN 111592525 B | 08 December 2020 |
| | | CN 111592525 A | 28 August 2020 |
| | | US 10844079 B2 | 24 November 2020 |
| | | US 2020317695 A1 | 08 October 2020 |
| | | EP 3712151 A4 | 30 December 2020 |
| | | CN 111566104 A | 21 August 2020 |
| | | EP 3712151 A1 | 23 September 2020 |
| CN 111153901 A | 15 May 2020 | WO 2020094104 A1 | 14 May 2020 |
| WO 2020072656 A1 | 09 April 2020 | US 2020108071 A1 | 09 April 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015107493 A1
- WO 2016203404 A1
- WO 2016203406 A1
- WO 2017216706 A1
- WO 2017211303 A1
- CN 201710062495
- WO 2018136265 A1
- WO 2018057884 A
- WO 2020094018 A


### Non-patent literature cited in the description


- **CAREY ; SUNDBERG.** ADVANCED ORGANIC CHEMISTRY 4TH ED. Plenum Press, 2000, vol. A
- Chiral Separations, Methods and Protocols. Methods in Molecular Biology. 2004, vol. 243
- **A.M. STALCUP.** Chiral Separations. *Annu. Rev. Anal. Chem.,* 2010, vol. 3, 341-63
- VOGEL'S ENCYCLOPEDIA OF PRACTICAL ORGANIC CHEMISTRY. Longman Scientific and Technical Ltd, 1991, 809-816
- **HELLER.** *Acc. Chem. Res.,* 1990, vol. 23, 128
- **GOODMAN ; GILMAN.** The Pharmacological Basis of Therapeutics. Pergamon
- Remington's, Pharmaceutical Sciences. Mack Publishing Co,
- **GREENE, T. W. ; P. G. M. WUTS.** Protective Groups in Organi Synthesis. Wiley, 1999